# EUROPEAN PATENT APPLICATION

(11) **EP 2 500 336 A1**
(43) Date of publication of application: **19.09.2012**
(21) Application number: 09851317.9
(22) Date of filing: 13.11.2009
(51) Int. Cl.: C07C 50/04

(54) **PHARMACEUTICAL SUBSTANCES ON THE BASIS OF MITOCHONDRIALLY ADDRESSED ANTIOXIDANTS**

(71) Applicant: Obschestvo S Ogranichennoi Otvetstvennostyu "Mitotekh", Moscow 117218 (RU)
(72) Inventor: SKULACHEV, Maxim Vladimirovich, Moscow 119234 (RU); SKULACHEV, Vladimir Petrovich, Moscow 119234 (RU); ZAMYATIN, Andrei Alexandrovich, Moscow 119334 (RU); EFREMOV, Evgeny Stepanovich, Moscow 117418 (RU); TASHLITSKY, Vadim Neronovich, Moscow 107207 (RU); YAGUZHINSKY, Lev Sergeevich, Moscow 123154 (RU); KORSHUNOVA, Galina Anatolievna, Moscow 117133 (RU); SUMBATYAN, Natalya Vladimirovna, Moscow 119602 (RU); ANTONENKO, Yury Nikolaevich, Moscow 119313 (RU); SEVERINA, Inna Isaakovna, Moscow 119234 (RU); CHERNYAK, Boris Viktorovich, Moscow 119602 (RU)
(74) Representative: Richards, John
(86) International application number: PCT/RU2009/000621
(87) International publication number: WO 2011/059355

(57) **Abstract**

This invention relates to the fields of pharmaceutics and medicine, and, in particular, relates to the production and use of pharmaceutical substances on the basis of mitochondria-addressed compounds. The invention discloses methods for synthesis, purification and storage of mitochondria-addressed antioxidants, making it possible to produce said substances in a form and quality meeting the demands made on active ingredients of medicinal preparations - the pharmaceutical substances. The invention also discloses methods for design and selection of new mitochondria-addressed antioxidants having specified properties.

## Description

### Field of the invention

This invention relates to the fields of pharmaceutics and medicine, and, in particular, concerns the production and use of pharmaceutical substances on the basis of mitochondria-addressed compounds.

### Background of the invention

Data published to date clearly demonstrate good pharmaceutical perspectives of a new class of biologically active substances ― mitochondria-addressed antioxidants (MAA) (see Skulachev V.P. (2005), IUBMB Life., 57:305-10; Skulachev V.P. (2007) Biochemistry (Mosc)., 72:1385-96; Antonenko Yu.N. et al. (2008), Biochemistry (Mosc).,73:1273-87, Skulachev V.P. et al., (2009), Biochim Biophys Acta., 1787:437-61, Smith R.A. et al., (2008), Ann. N. Y. Acad. Sci., 1147:105-11, see also WO2007046729, WO2009005386, US 6331532, EP 1047701, EP 1534720) on the basis of 'Skulachev-ions' (the term was coined by Green D.E., "The electromechanochemical model for energy coupling in mitochondria", 1974, Biochem. Biophys. Acta., 346:27-78).

The above mentioned sources disclose the results of studies of MAA under laboratory conditions - *in vitro* or in animal models. However in order to use any compound as active pharmaceutical ingredient (so-called pharmaceutical substance), the compound must meet certain requirements, namely:
1. Comply fully with the national regulators requirements summarized in corresponding documents, pharmacopoeial monographs. The main requirements are: authenticity, impurity content, heavy metal content, water content, residual organic solvent content, sterility, method of quantative measurement of the compound, methods of packaging, labeling and transportation.
2. Characteristics of the compound listed in regulatory documents as well as pharmaceutical activity must remain within postulated limits during the postulated shelf storage time.

Particular attention should be drawn to the total content of impurities, as well as the content of single impurities. In particular, single impurities which cannot be individually identified and fully characterized should not constitute a significant proportion (in most cases - more than 1%) of the total content of impurities.

Another significant difficulty with practical applications of MAA as pharmaceutical substances is the fact that in the descriptions of inventions related to MAA (see above) a large number of compounds claimed as mitochondria-addressed antioxidants have been disclosed. However, the results of the experiments (for example, see Antonenko Yu.N. et al. (2008), Biochemistry (Mosc).,73:1273-87) including clinical trials show that the disclosed mitochondria-addressed compounds have different (sometimes even opposite) biological activity. In this regard, development of methods for design of a biologically active substance with well-defined, predetermined properties suitable for the specific application of the compound remains urgent. It is also urgent to predict properties and biological activity (in the first place - clinical activity) of MAA on the basis of Skulachev-ions.

### Definitions

**Pharmaceutical substance ―** a substance which is prepared for use as ngredient of a medicinal preparation and meets the pharmacopoeia requirements.
**Skulachev-ions** - lipophilic cations and anions which are able to penetrate through the mitochondrial membrane.
**Mitochondria-addressed antioxidants (MAA)** - compounds which can targetedly accumulate into mitochondria and possess antioxidant activity.

### Description of the invention

Aspects of the invention are given below:
**I.** The present invention is devoted to the production of pharmaceutical substances on the basis of mitochondria-addressed antioxidants (MAA), the design and selection of specific MAA, which best correspond to relevant clinical tasks. In particular, the invention relates to MAA compounds comprising an antioxidant that is attached through a linker group to a lipophilic cation ('Skulachev-ion'). These MAA can be described by general formula (I) given below:
   **General formula (I):** compounds of structure 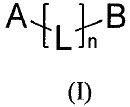 wherein **A** is effector moiety; **L** ― linker group, n - integer from 1 to 20; **B** ― targeting group that provides targeted delivery of the compound into mitochondria.
      Wherein **A** may be an antioxidant of general formula (II) 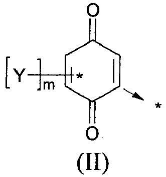 and/or reduced form thereof
      wherein **m** - integer from 1 to 3; **Y** - identical or different substituents selected from lower alkyl or lower alkoxy; or two vicinal Y are connected to each other so that they form a structure (III): 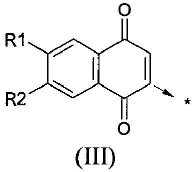 and/or reduced form thereof
      wherein R₁ and R₂ are identical or different substituents selected from lower alkyl or lower alkoxy;
**L** ― linker group, comprising:
   a) either straight or branched hydrocarbon chain optionally containing one or more double or triple bond, or ether bond, or ester bond, or C-S, or S-S, or peptide bond; and which is *optionally* substituted by one or more substituents preferably selected from alkyl, alkoxy, halogen, keto group, amino group;
   b) or natural isoprene chain;
**B** ― targeting group comprising:
   a) either Skulachev-ion Sk:

      Sk⁺ Z⁻

      wherein Sk ― lipophilic cation;
      Z - pharmaceutically acceptable anion;
   b) or amphiphilic zwitterion which is able to penetrate into mitochondria in its cationic form.
   c) In addition, Sk⁺ as a component of **B** may also be a lipophilic metal-organic compound, in particular, lipophilic metalloporphyrin preferably having a structure: 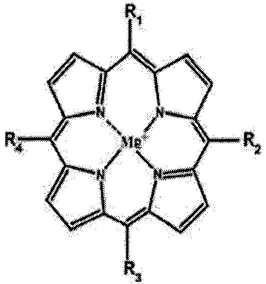 included in the composition of a compound of formula (I) through the moieties designated R₁, R₂, R₃ or R₄. Wherein the remaining substituents R₁, R₂, R₃ or R₄ may be selected in accordance with required properties of the compound, in particular, in order to increase or decrease hydrophobicity of the molecule; Me⁺ denotes a metal ion preferably selected from Mn, Fe, Co, Cu, Mg or Zn.

Examples of structures of biologically active compounds belonging to MAA of general formula (I) are shown in a scheme below.

SkQ1 (plastoquinonyl-decyl-triphenylphosphonium (PDTP) bromide)

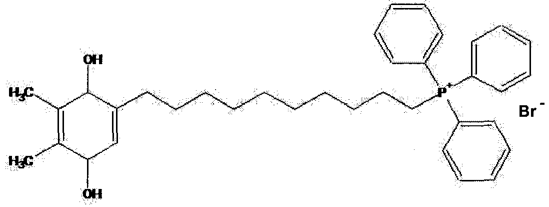

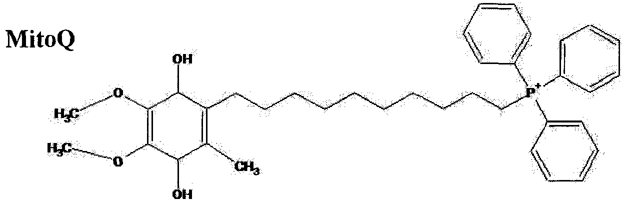

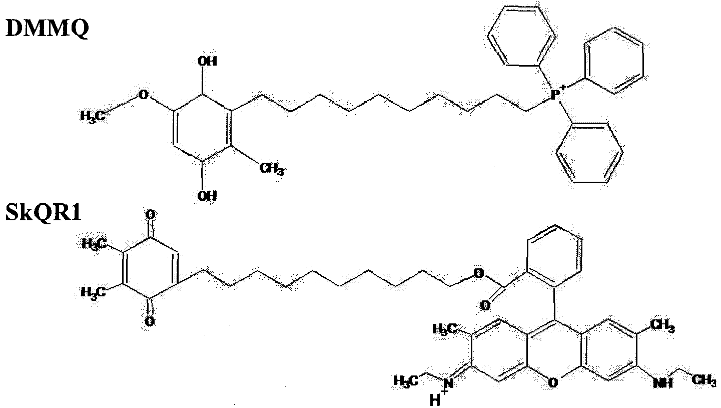

In some cases, in an embodiment of the present invention, it is advisable to use compounds of formula (I) wherein pro-oxidants are used as effector moiety A, in particular, desmethoxyubiquinone or ionol described by the following structures:

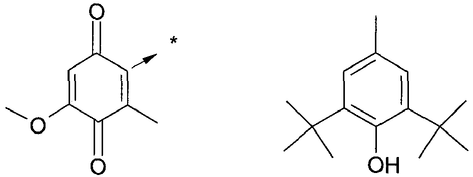

The corresponding compounds of formula (I) will be mitochondria-addressed pro-oxidants.

### II. Methods and approaches for selection

Another aspect of the present invention is a method of designing and/or selection of a specific mitochondria-addressed compound.

### II.a Method of designing new mitochondrial antioxidants

The study of chemical, physicochemical and biological properties of said compounds allowed to propose a new approach for design of compounds belonging to a class of compounds (I). Using the proposed model it is possible to design a structure of new mitochondria-addressed antioxidants with predetermined properties.

A scheme of design of compounds describing in general the proposed design model of the compounds of the invention is shown in a scheme below:

### Scheme of design model of new mitochondria-addressed antioxidants

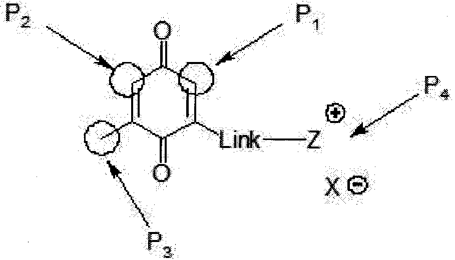

wherein:
P1 - a position that is responsible for stability and biological activity of a compound. If this carbon atom has no substituent, such a substance is maximally efficient as an antioxidant but relatively unstable. SkQ1 which is 10 times more potent antioxidant than MitoQ is an example. However, the presence of a methyl group at position P1 in MitoQ makes it more stable as compared to SkQ1. The composition of the linker 'Link' can also affect the stability of a substance. For example, the stability of a substance can be changed with introduction of ester bond, peptide bond, sulfide group or other reactive groups to 'Link'.

Positions P2 and P3 are responsible for regulation of the interaction with the mitochondrial respiratory chain. If one of these carbon atoms has no substituent, such a substance cannot be reduced with the mitochondrial respiratory chain due to which the compound is converted into a pro-oxidant. The same effect can be achieved if the structure of one or both of the substituents at this position does not allow the respiratory chain to reduce and/or oxidize a corresponding compound.

Substituents at the same positions P2 and P3 may affect the ratio between pro-oxidant and antioxidant properties of the compound. The presence of oxygen atoms at positions P2 and P3 may lead to the formation of internal hydrogen bond with the hydrogen atom of OH group of quinol in the reduced or partially reduced (quinol or semiquinone) forms of an antioxidant. Such hydrogen bond may hinder the oxidation of OH group in the reaction with free radicals and reactive oxygen species that drastically reduces the antioxidant properties of a substance as compared to a compound in which there are no oxygen atoms at positions P2 and P3 (for example, there are methyl groups). This may explain the difference between these properties in SkQ1 and MitoQ.

P4 - a position that is responsible for penetrating ability of a biologically active substance. The ability to penetrate into mitochondria depends on charge and hydrophobicity of a compound. For example, experiments on artificial membranes show that compounds with triphenylphosphonium at position P4 are less penetrating than substances where at that position there is more hydrophobic cation - rhodamine G moiety.

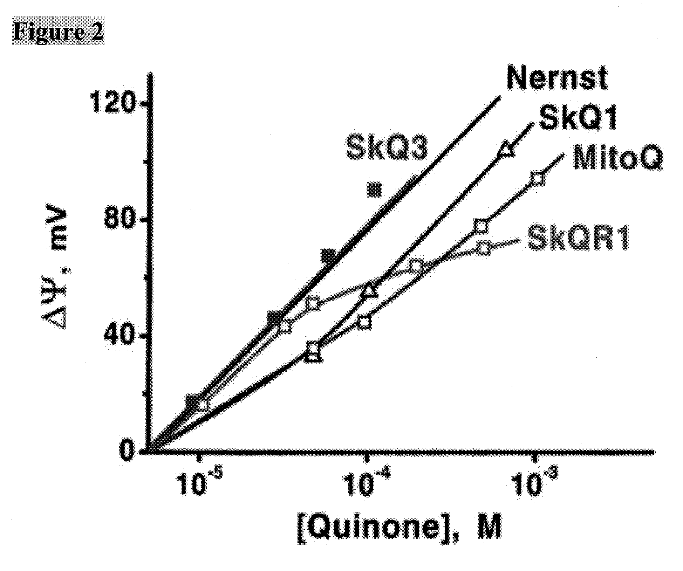

'Link' - the structure element that is also able to dramatically affect the properties of the compound. The length and composition of the linker 'Link' may affect the penetrating ability of the compound (Fig. 2). Reducing the length of the linker and increase in its hydrophilicity will reduce the penetrating ability of the compound. Also modifying the composition of the linker 'Link' one can change the stability of the compound - introduction of ester bonds, peptide bonds, other bonds which are less stable than the C-C bond into the linker can make the compound vulnerable for cellular enzymes such as esterases, peptidases. Also changing the length of this element one can change the position of the hydrophobic part of the molecule (antioxidant moiety) within the bilayer membrane that is an important factor in determining a possibility of interaction of the compound with the mitochondrial respiratory chain.

Thus, one aspect of the present invention relates to a method for creating structures (design) of mitochondria-addressed compounds with predicted biological activity. The biological activity is defined as the influence on biological systems and their models (i.e., artificial cell-free systems, subcellular fractions and organelles, cells, regions of tissues and organs or whole organism), that comprises antioxidant effect, pro-oxidant effect, uncoupling effect on mitochondria, a change in the properties of biological membranes, regulatory effect through different messengers at different levels (regulation of gene expression, regulation of protein activity, regulation of hormonal profile of an organism etc.).

### II.b Method to design compounds using combinatorial libraries

Another aspect of the present invention relates to a combinatorial library of mitochondria-addressed compounds and methods for search and selection of promising compounds from this library. Said library is a set of compounds of general formula (I) which are actually able to targetedly accumulate into mitochondria. The compounds for the library may be synthesized including on the basis of the general part that is a lipophilic cation connected to a linker (part of the linker) bearing an 'activated' residue, for example, halogen through which the attachment of a variable part of the compound occurs. In other words, the library of mitochondria-addressed compounds can be obtained by attaching non-addressed low molecular weight compounds to the lipophilic cation of the library.
**II.c** Another aspect of the present invention is a method of testing (including if the method is automated or semi-automated) for the biological activity of compounds of the library in order to select compounds with desired activity. Said method comprises the following steps:
   1) test that allows to select a group of candidate substances from the library;
   2) construction of a combinatorial sublibrary based on the selected substances and their modifications if any;
   3) testing sublibrary to select compounds with the most pronounced desired biological activity;
   4) repeat steps 1 to 3 until all possible variants of the compounds are tried or until the desired biological activity is achieved.

   It should be noted that a combination of several methods for testing the biological activity at steps 1 and 3 that can significantly reduce the probability of artifact results is the most effective. The specific test methods can be adapted by qualified experts in the field of biochemistry, biophysics, bioenergetics, microbiology, molecular biology, cell biology or other fields of modern biology on the basis of publicly available literature data on the methods ofwork with combinatorial libraries and methods listed in the description of the invention (see sections ***test methods, methods for interpretation of results, experimental examples).*** The experimental examples given in the appropriate section are the methods for testing the activity of mitochondria-addressed compounds 'in individual test tubes' and can be easily adapted for testing combinatorial libraries by highly-productive methods using standard approaches.

### III. Test methods

Another aspect of the present invention is a set of test methods used to test the biological activity of new mitochondria-addressed compounds of general formula (I). The test compounds can be studied both individually and as part of combinatorial libraries. Said set of test methods comprises the following methods:
1) testing redox properties and stability of the compounds of general formula (I) *in vitro;*
2) testing penetrating ability of mitochondria-addressed compounds on artificial black membranes;
3) testing protective or damaging effect of mitochondria-addressed compounds on membrane proteins using artificial model membranes containing gramicidin channels;
4) testing antioxidant or pro-oxidant effect of mitochondria-addressed compounds on isolated mitochondria;
5) testing antioxidant or pro-oxidant action of mitochondria-addressed compounds in animal, plant, bacterial or yeast cell cultures;
6) testing anti-apoptotic or anti-necrotic or pro-apoptotic or pro-necrotic acitivity of mitochondria-addressed compounds in cell cultures;
7) testing accumulation of mitochondria-addressed compounds in cells;
8) testing specific activity of mitochondria-addressed compounds, said specific activity is defined as the ability to activate or suppress certain metabolic pathways that in turn may be manifested in activation of certain genes at the transcriptional level, mRNA stability or translation, at the level of protein modifications comprising phosphorylation or dephosphorylation, proteolysis, glycosylation, carbonylation and other ways of changing the activity of proteins or protein complexes; activation or inhibition of metabolic pathways may also be manifested in change of other physiological parameters of cells such as: change in the respiratory rate, change in the rate of production of certain metabolites, change in the rate of consumption of certain substrates, change in membrane potential on the outer membrane, on the mitochondrial membrane or membranes of other organelles, change in the ionic conductivity of one or more of said membranes, change in concentrations of certain ions including change in pH in cellular cytoplasm or in other cellular compartments, change in intracellular transport of biomolecules, vesicles and organelles, change during the cell cycle, change that leads to cell division, cell transformation, cell death or, conversely, to their survival;
9) testing biological activity of compounds of general formula (I) ***in vivo*** in animals or plants; said tests are not applicable at the step of initial testing combinatorial libraries and may only be applied to the study a limited number of compounds.

Thus, aspects of the present invention are each of said tests applied to study the properties of new compounds of general formula (I) including for forecast of prospects of said compounds. Said prospects are defined as prospects for practical use of said compounds in medicine, biotechnology, cosmetology.

### IV. Methods for interpretation of results

Another aspect of the present invention is a method for interpretation of results obtained during testing the compounds of general formula (I) in order to forecast the prospects of practical use of candidate compounds in medicine, biotechnology, cosmetology and in order to select the most promising compounds.

The main element of said method is to compare the results of testing candidate compounds with the results of testing compound SkQ1 which are listed in the section ***experimental examples**.* Thus, data on SkQ1 may be considered as the starting point, fixed point, the standard for predicting the effectiveness of other compounds of general formula (I), since SkQ1 is a quite effective biologically active compound and can be applied in medicine, biotechnology, cosmetology (see the patent applications PCT/RU2006/000394, PCT/RU2006/000646, PCT/RU2006/000547 and other publications related to SkQ1).

Compounds SKQR1, MitoQ, MitoVitE, DMMQ can serve as other standards for comparison of activity. A possibility for the test compound to be reduced with the mitochondrial respiratory chain is preferred, preferably with the enzymes and coenzymes of the respiratory chain, and a significant domination of antioxidant properties over pro-oxidant properties in the test compound.

Thus, compounds MitoVitE and DMMQ are a kind of 'negative' fixed points and upon selection of the most promising compounds ― antioxidants, compounds which have similar biological activity (starting from the level of testing on mitochondria) should be avoided. Compound MitoQ is also a 'negative' fixed point.

Upon selection of new compounds based on tests of their properties, one should select those compounds which in their activity are closer to SkQ1 than MitoQ. In the first place, the activity is defined as the ability to show antioxidant properties at low and ultra-low concentrations. Mitochondria-addressed antioxidants with increasing their doses have a strong pro-oxidative effect on various biological objects (see ***experimental examples,*** and also Doughan A.K., Dikalov S.I. (2007), Antioxid. Redox Signal. 9:1825-36). At lower doses, these compounds exhibit antioxidant properties.

Thus, the most important characteristic of compound - mitochondria-addressed substance is the so-called 'window of application', i.e., the difference between minimum concentration (dose) of a substance that already displays antioxidant properties and minimum concentration (dose) of a substance manifesting pro-oxidant properties. It is obvious that exceeding the latter concentration (dose) is extremely undesirable in practical application of the substance and may significantly limit the possibility of such an application. Methods for assessing the 'window of application' at different levels are given in the section ***experimental examples.*** A pair of compounds SkQ1 - MitoQ can serve as a good fixed point for assessing the prospects of the test compounds, since SkQ1 has sufficient 'window of application' while MitoQ has not.

A method for interpretation of results of testing compounds is given below. The method is divided into several stages:
1) on the bases of the results of testing properties of substances *in vitro* (tests 1,2,3 of the section ***Test methods)*** one can estimate the antioxidant and pro-oxidant properties of test compounds; this allows to predict their applicability in the fields where the use of antioxidant or pro-oxidant is useful; *in vitro* tests also allow to determine the ability of candidate compounds to penetrate through biological membranes and thus predict bioavailability of the compounds, their ability to overcome various barriers in an organism (e.g., blood-brain barrier) as well as their stability;
2) examples of interpretations of test results which are an aspect of the present invention, are given in the section ***experimental examples;*** on the basis of said examples and corresponding interpretations a qualified expert in the field of biochemistry, biophysics, bioenergetics, microbiology, molecular biology, cell biology or other fields of modem biology can correctly assess the results of testing candidate compounds and select the most promising and the most suitable compounds for the required practical application.

To confirm the feasibility of the present invention and the correctness of the proposed model, new mitochondria-addressed antioxidants SkQ3, SkQ4, SkQ5 and SkQB1 have been synthesized.

### V. Examples of compounds whose prospects are predicted by the present invention:

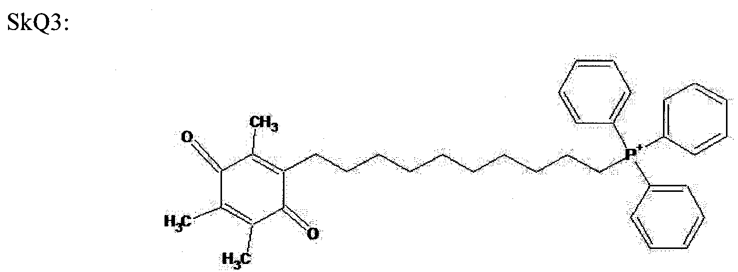

Predicted specifications: the compound must be more stable but have less pronounced antioxidant properties as compared with SkQ1. The compound can be used in plant biotechnology, mycology and microbiology.

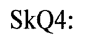

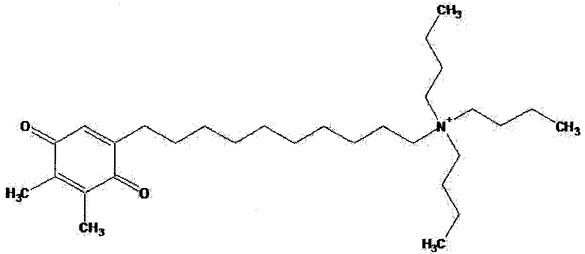

Specifications: lower (as compared with SkQ1) ability to penetrate through biomembranes. Thereby bioavailability of the preparation and severity of side effects must be reduced.

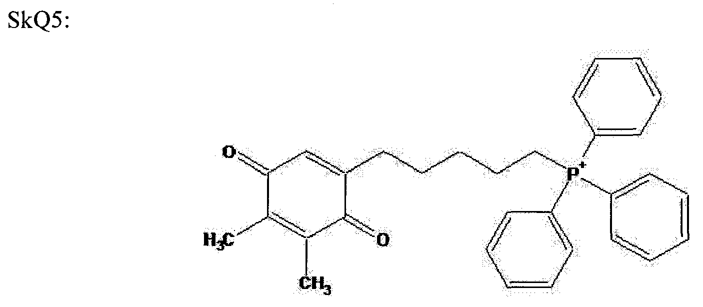

Specifications: decrease in the length of the linker between 'Skulachev-ion' and antioxidant reduces hydrophobicity of the compound and can affect the rate of penetration of the compound through the membranes.

A series of SkQB compounds - with enhanced penetrating ability as compared with SkQ1. Said series comprises all the compounds in which natural compounds, for example berberine, are used as 'Skulachev-ion'. Compounds of the SkQB series (e.g., SKQB1 whose formula is given below) have enhanced penetrating ability and therefore are more promising from the pharmaceutical point of view, they have a greater ability to overcome the blood-brain barrier and blood-ophthalmic barrier. Also, compounds of the SkQB series should have less severe side effects, since berberine (as well as palmatine) are natural compounds of plant origin.

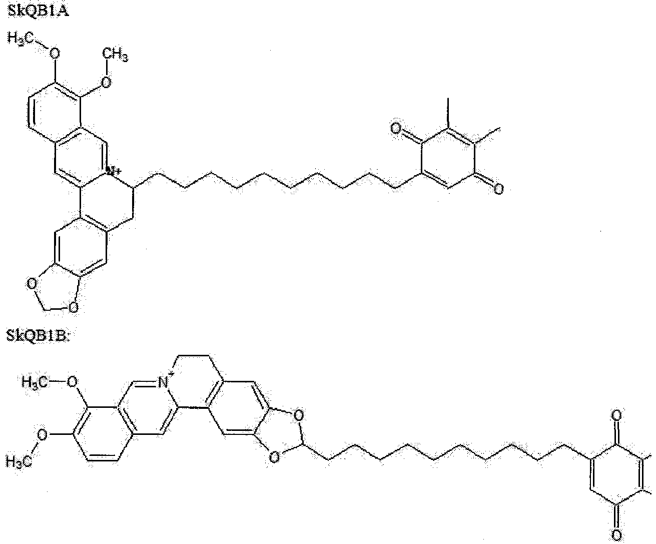

In general, SkQB based on berberine may be represented by general formula: 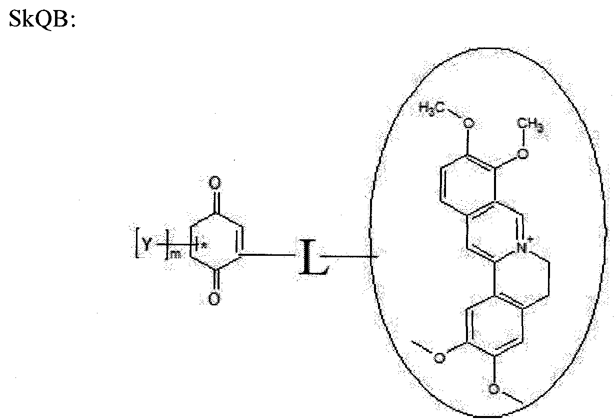 wherein **m** - integer from 0 to 3 (preferably - 2, i.e., the left side of the formula is plastoquinone moiety), '**L**' - linker that has the length from 1 to 50 units comprising:
1) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
2) natural isoprene chain.

Preferably, 'L' is decane moiety. The right side of the compound formula is berberine moiety attached to linker 'L' through one of its constituent atoms. The attachment can be made through C-C, C-O, C-N, C-S bonds including ester bond, peptide bond, disulfide bond. Including the attachment can be made through ether bond by displacing one of the methoxy groups of berberine.

In compounds of the SkQB series, palmatine may be used in place of berberine.

Also preferred compounds on the basis of berberine and palmatine are the following compounds:

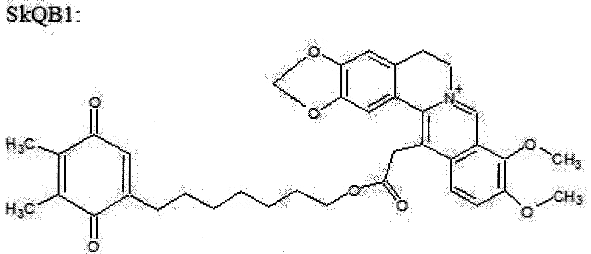

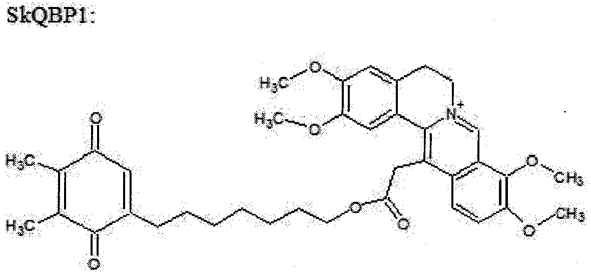

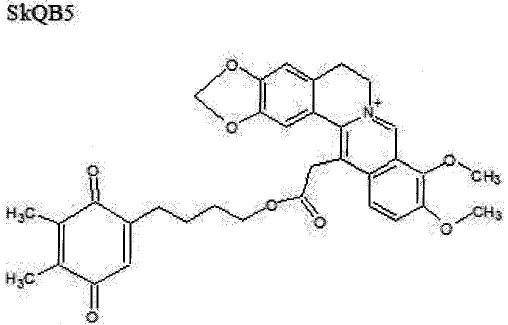

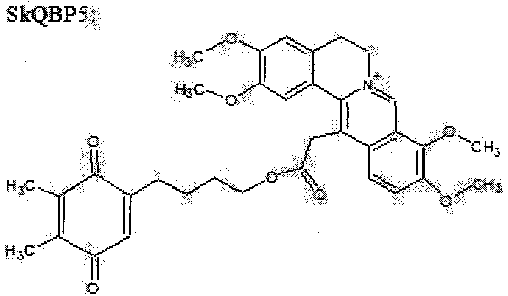

Methods of synthesis and description of the chemical properties and biological activity of said compounds are given in the section ***experimental examples.***

### VI. Pharmaceutical substances on the basis of mitochondria-addressed antioxidants.

One aspect of the present invention is parameters of regulatory documents which allow to use MAA-based pharmaceutical substance in medical practice and, in particular, comprising the following indicators:
1. authenticity determined in particular by means of
   a. spectrophotometry at a given wavelength range and comparison with the results of a spectrophotometric study of the MAA sample
   b. IR spectroscopy. The infrared spectrum of a substance taken by the method of Frustrated Total Internal Reflection must have coincidence of the absorption bands with the absorption bands of the included spectrum in position and intensity of the bands.
   c. Reaction to bromides. The chloroform layer should turn yellow.
2. Impurity content determined by HPLC method. The content of each individual impurity does not exceed 1.5%. Total impurity content does not exceed 4.0%.
3. Heavy metal content (does not exceed 0.001 %).
4. Residual organic solvent content (such solvents as ethanol, methanol and chloroform).
5. Sterility.
6. Quantification of the substance
7. Packaging, labeling and storage
8. Established expiration date

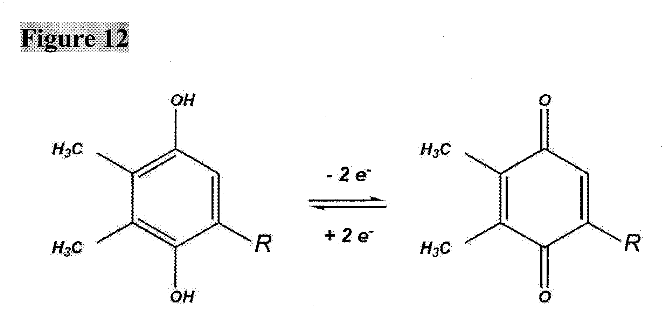

In practice, the known quinone-containing mitochondria-addressed compounds including substances SkQ1, SkQR1, MitoQ and others (see above) are mainly in the one oxidized (quinone) form. Under normal conditions, the oxidized form can be partially reduced to the quinol form (see Fig. 12). Thus, when using MAA as pharmaceutical substances, the main impurity whose content can reach 5-8% and more is the reduced form of MAA. Isolation and detailed study of this form is a difficult task because of its high chemical lability and tendency to undergo complex redox transitions.

On the other hand, production of a pharmaceutical substance MAA in the quinone form free of the reduced (quinol) form by standard methods for chromatographic purification is difficult because of the similarity of these compounds.

The second problem encountered in the purification process of the said series of MAA preparations is the need to preserve a characteristic counterion, for example, a bromine ion. Usual conditions for ion exchange or high performance liquid chromatography (HPLC) are too harsh with respect to said labile compounds and do not guarantee preservation of the counterion in initial structure.

Thus, existing methods and techniques for isolation and purification of preparations of MAA series do not provide necessary parameters of purity for such type of pharmaceutical substances.

In the present invention said problem can be solved by:
Method 1. The use of non-standard HPLC in salt-free un-buffered mobile phase system and at the final stage - gel-filtration of highly concentrated solution of the preparation.
Method 2. The use of 'molecular trap' for the reduced form of MAA in a form of an agent in a nonpolar solvent which acts as an effective inducer of oxidation or a competitive substituent of the quinone form in the quinone-quinol equilibrium.

Thus, another aspect of the present invention is a method of producing MAA in a form suitable for pharmaceutical substances and comprising Method 1 and/or Method 2. VII. Another aspect of the present invention is an improved method for synthesis of MAA on the basis of quinones. The improved method allows for the industrial production (synthesis) of MAA using cheaper and more available components, in particular, in the case of the synthesis of plastoquinonyl-decyl-triphenylphosphonium (PDTP) bromide and other derivatives of plastoquinone, 2,3-dimethylphenol rather than dimethyl hydroquinone can be used as initial reagent.

The synthesis involves the following steps:
1. Oxidation of 2,3-dimethylphenol (1) to 2,3-dimethyl-1,4-benzoquinone (2) with the Jones reagent.
2. Attachment of 11-bromo-undecanoic acid (3) to triphenylphosphine with the formation of(10-carboxy-decyl)triphenylphosphine bromide (4).
3. Formation of the desired compound (5) by the reaction of the produced compound (4) with 2,3-dimethyl-1,4-benzoquinone (2) in the presence of silver nitrate and ammonium persulfate.

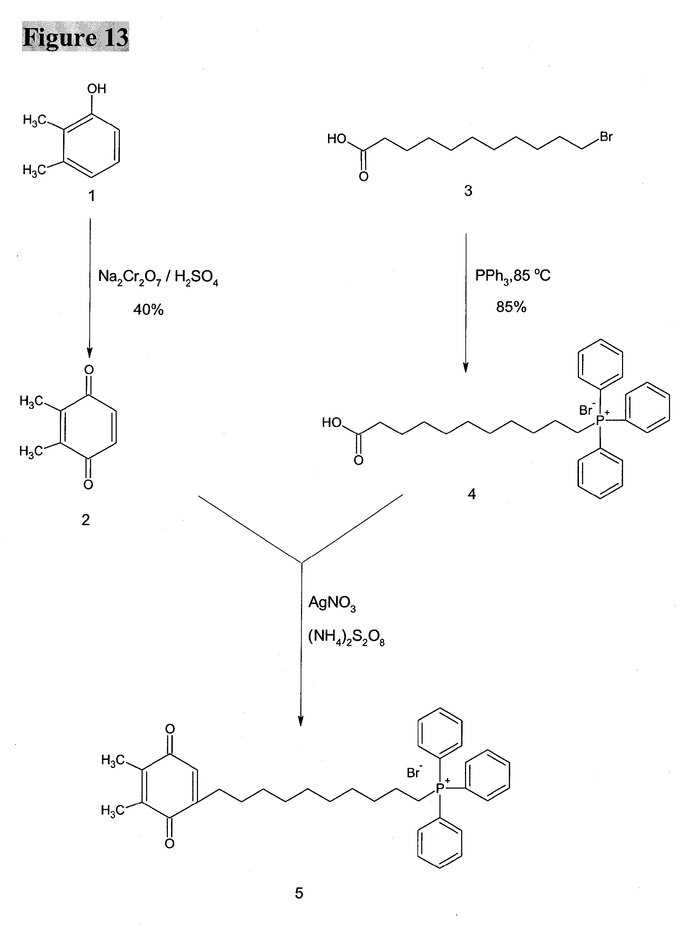

The general scheme of the synthesis is shown in Figure 13. In more detail, the synthesis is described in the ***Experimental example 8.***

### Brief description of figures

**Figure 1****.** The ability of compounds of general formula (I) to be oxidized by oxygen (A) or superoxide (B) formed by the reaction of xanthine oxidase with xanthine.
**Figure 2****.** Comparison of the ability of compounds of structure (I) to pass through the bilayer membrane and form a membrane potential. For comparison, estimates for ideally penetrating monocation (according to the Nernst equation) are shown by black line.
**Figure 3****.** Testing protective or damaging effect of mitochondria-addressed compounds on membrane proteins using artificial model membranes containing gramicidin channels. Damage to gramicidin channels was stimulated by photoactivation of phthalocyanine photosensitizer (A) or a mixture of FeSO₄, ascorbate and tert-Butyl hydroperoxide (B).
**Figure 4****.** Accumulation of SkQ1 and SkQ5 in mitochondria measured with the use of the TPP⁺ electrode.
**Figure 5****.** The ability of mitochondria to reduce or oxidize SkQ1 depending on the activity of respiratory chain components.
**Figure 6****.** SkQ1 protects mitochondria under conditions of oxidative stress caused by a mixture of ferrous sulfate with potassium ascorbate.
**Figure 7****.** SkQ1 and MitoQ protect cells against death induced by H₂O₂ (300 µM). Cells were incubated with SkQ 1 or MitoQ for 7 days and then were seeded for the experiment.
**Figure 8****.** Short pre-incubation of HeLa cells with SkQR1 (2 hours) reduces the levels of oxidative stress in cells induced by H₂O₂ (300 **µ**M). Cytofluometry data (A) on the basis of which the number of cells with low levels of oxidative stress was estimated (B). Estimates were made on the basis of seven experiments. Data were estimated with respect to a group of the control cells lying on the diagram in the area of low DCF-DA fluorescence (50% of the population), this group of cells was taken as 100% and indicators for every action were estimated relative to this group.
**Figure 9****.** Treatment of cells with SkQ1 (20 nM) for 7 days protects HeLa cells against oxidative stress induced by H₂O₂ (300 **µ**M).
**Figure 10****.** Luminescence induction in *E. coli* MG1655 pLUX::PsoxS in the presence of hydrogen peroxide (100 **µ**M) and paraquat (100 **µ**M).
**Figure 11****.** Induction of soxS promoter by hydrogen peroxide (500 **µ**M) and paraquat (100 **µ**M) in the presence SkQ1 (10 **µ**M).
**Figure 12****.** Equilibrium of quinone and quinol forms of mitochondria-addressed antioxidants. R - linker group connected to lipophilic ion ('Skulachev-ion').
**Figure 13****.** Scheme for improved method of synthesis of mitochondria-addressed antioxidant PDTP.

### Experimental examples

The following are experimental examples intended to illustrate the possibility of applying the invention to the development of new mitochondria-addressed compounds. The results of experiments (tests) are also starting points for evaluating prospects for new compounds developed (or selected from combinatorial libraries) by experts in the field using the present invention to search for new mitochondria-addressed compounds. In this regard, experimental examples are called 'tests', since they are methods for testing new compounds.

### Experimental example 1. In vitro testing redox properties and stability of compounds of general formula (I).

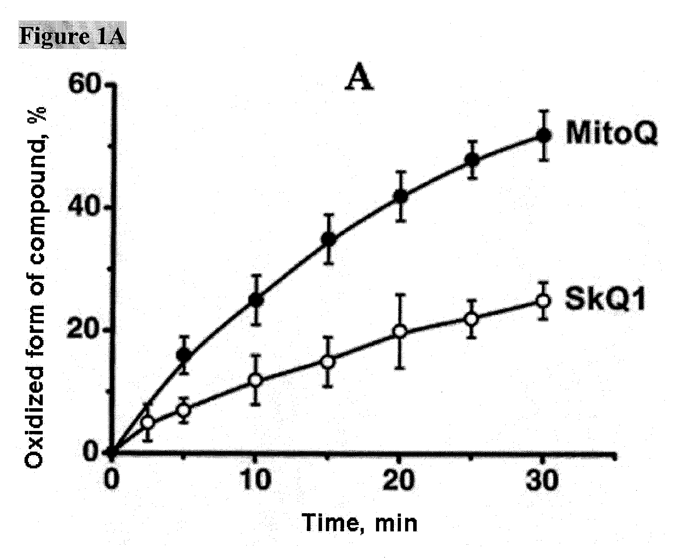

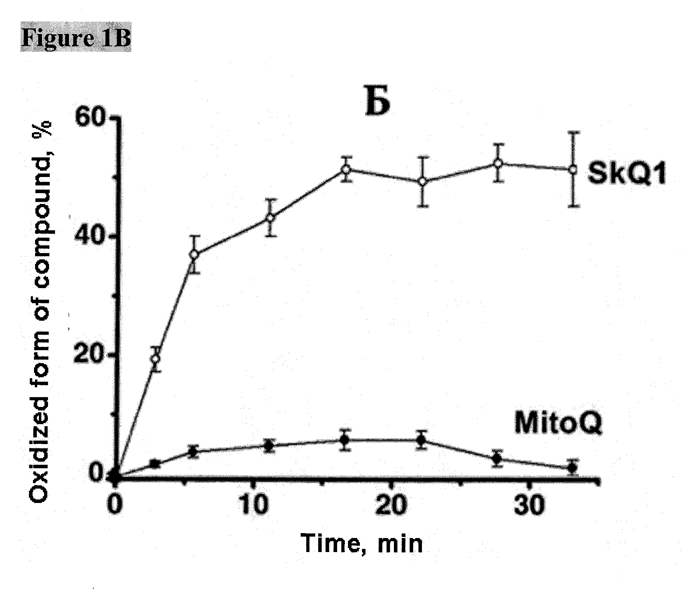

The first step in selection of compounds corresponding to structure (I) is testing their redox properties. Namely at this step one can select substances with predetermined pro-oxidant or antioxidant properties. The easiest way to select compounds with potential antioxidant properties is testing their ability to be oxidized by oxygen or superoxide formed in the reaction of xanthine with xanthine oxidase. Stability of the reduced forms of SkQ1 and MitoQ over time was investigated by analysis of absolute absorption spectra of said compounds in the range from 240 to 310 nm recorded using a double-beam Pye Unicam SP 1100 spectrometer (England). Under a developed method, quinone derivatives were reduced with sodium tetrahydroborate in a medium contained 20 mM MOPS-KOH, pH = 7.6. A reference cuvette contained neither SkQ1 nor MitoQ, the reductant was added to both cuvettes, measurements were made after the release of the hydrogen. The degree of reduction of quinones was assessed by the magnitude of the peak area by weighing method, the absolute value of the absorption maximum at 267 nm was measured for comparison. The data indicate that the reduced (quinol) form of SkQ1, when exposed to oxygen, is more resistant to oxidation by atmospheric oxygen as compared to MitoQ **(****Fig. 1A****)**. Thus, SkQ1 is less prone to interact spontaneously with oxygen and form radicals that potentially indicates it is less toxic for a cell. On the other hand, when the compounds were oxidized not by oxygen but by superoxide formed in the reaction of xanthine oxidase with xanthine, the oxidation of SkQ1 was much more effective than that of MitoQ **(****Fig. 1B**). This may indicate that SkQ1 is more potent as an antioxidant and its reduced (active) form is more resistant to spontaneous oxidation by atmospheric oxygen as compared to MitoQ and has a higher affinity for superoxide radical.

### Experimental example 2. Testing penetrating ability of mitochondria-addressed compounds on artificial black membranes.

To test penetrating ability of mitochondria-addressed compounds of structure (I), it is proposed to use a method based on the ability of ions to penetrate through the bilayer phospholipid membrane moving along the concentration gradient. The bilayer membrane separates two chambers filled with an aqueous solution and the test substance is added to one of the chambers. If a charged substance can penetrate through the bilayer membrane, its rapid diffusion out of the chamber with a high concentration of the substance to the chamber with a low concentration of the substance occurs and thus the membrane potential difference is created. For ions which are carrying one charge and able to easily penetrate through the membrane, a 10-fold concentration gradient allows for creation of a potential of 60 mV (according to the Nernst equation).

Said method was used in various studies on the ability of ions to pass through the lipid bilayer of the membrane and was described in detail in an article of Starkov AA, Bloch DA, Chernyak BV, Dedukhova VI, Mansurova SA, Symonyan RA, Vygodina TV, Skulachev VP, 1997, Biochem. Biophys Acta, 1318, 159-172. With said method, several substances of structure (I), such as SkQ1, SkQ3, SkQR1 and MitoQ, were tested. It was shown that SkQ3 and SkQR1 fully obey the Nernst equation at concentrations ranging from 5×10⁻⁶ to 5×10⁻⁴ M (for SkQ3) and from 5×10⁻⁶ to 5×10⁻⁵ M (for SkQR1). At concentrations higher than 5×10⁻⁵ M SkQR1 ceases to obey the Nernst equation that is probably due to its ability to damage the membrane at high concentrations. The SkQ1 and MitoQ gradient begins to create the potential in accordance with the Nernst equation at higher concentrations (ranging from 5×10⁻⁵ to 5×10⁻⁴ M). Thus, on the basis of the data, one can conclude that SkQ1 and MitoQ have less penetrating ability as compared to SkQ3 or SkQR1. This relatively simple method is ideal at the stage of primary selection of proposed mitochondria-addressed compounds of structure (I), as it allows to quickly select the compounds which have the highest penetrating ability, i.e., potentially are more bioavailable.

The penetrating ability of compounds SkQB1 and SkQBP1 was also analyzed. The results showed high penetrating ability of said compounds (their ability is not inferior to that of SkQ1).

### Experimental example 3. Testing protective or damaging effect of mitochondria-addressed compounds on membrane proteins using artificial model membranes containing gramicidin channels.

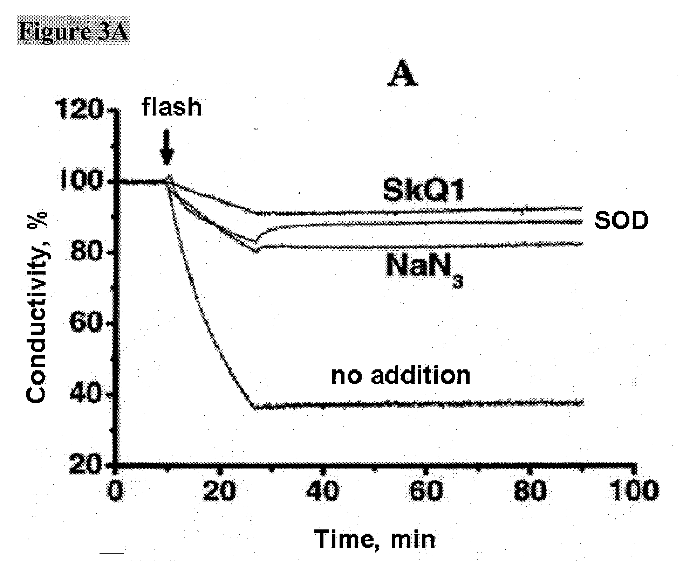

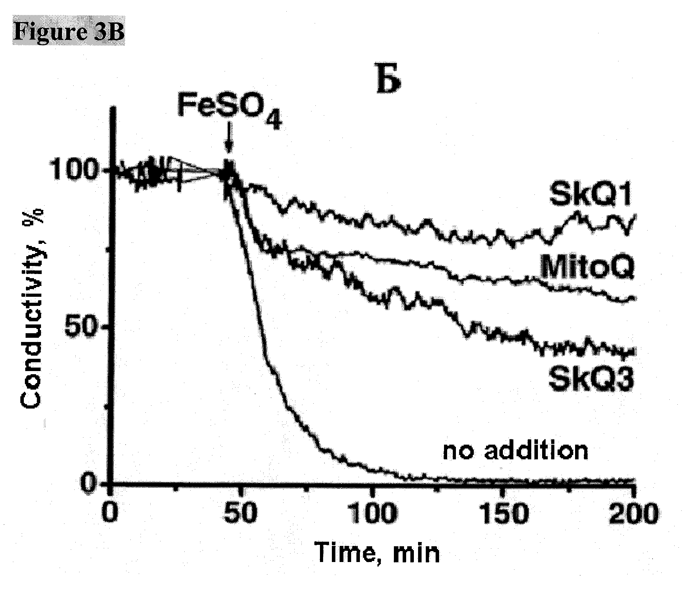

In our laboratory, a method which allows to study the antioxidant activity of compounds in a simple system consisting of a bilayer membrane, a conducting protein gramicidin and a photosensitizer (Mito Tracker Red, thrice sulfonated aluminum phthalocyanine or zinc phthalocyanine) was developed. The method consists in the ability of reactive oxygen species generated by photoactivation of the photosensitizer molecules to damage gramicidin channels resulting in a sharp decrease in the conducting ability of the bilayer membrane. Apart from photosensitizers, damage to gramicidin channels can be induced by initiating the Fenton reaction resulting in the formation of such highly reactive oxygen species as the hydroxyl radical (the Fenton reaction is initiated by a mixture of FeSO₄, ascorbate and tert-Butyl hydroperoxide). With said method, compounds SkQ1, SkQ3 and MitoQ were tested. **Figure 3A** shows that photoactivation of phthalocyanine by a short flash of light leads to a strong decrease in membrane conductance due to damage to gramicidin channels in the bilayer membrane. The fall in membrane conductance was effectively blocked by sodium azide (a highly efficient system to trap singlet oxygen) as well as by the enzyme superoxide dismutase (it catalyzes the conversion of superoxide into relatively low active hydrogen peroxide). Neither sodium azide nor superoxide dismutase prevents completely the fall in conductance of the bilayer membrane. This fact indicates that photoactivation of phthalocyanine is associated with generation of both singlet oxygen and superoxide. In this model, SkQ1 was the most efficient, since SkQ1 is a broad-spectrum antioxidant that protects against various reactive oxygen species. In another model, where damage to gramicidin channels was stimulated by a mixture of FeSO₄, ascorbate and tert-Butyl hydroperoxide, SkQ1 was also the most efficient, while MitoQ and SkQ3 appeared to be less efficient antioxidants **(****Figure 3B****).**

The method used for testing the antioxidant capacity of the synthesized compounds is a highly efficient and allows not only to evaluate the antioxidant activity of the compounds but also to determine specificity of the compound towards specific reactive oxygen species. As a reference substance for said method, it would be correct to use SkQ1 as the most efficient compound exhibiting the antioxidant activity towards a wide range of reactive oxygen species.

### Experimental example 4. Testing antioxidant or pro-oxidant effect of mitochondria-addressed compounds on isolated mitochondria.

There are a number of methods where the object of research is mitochondria. We have selected the most informative methods which with a high degree of certainty allow to determine a potential bioactivity of mitochondria-addressed compounds of general formula (I).

### i) Testing ability of compounds of general formula (I) to accumulate in mitochondria.

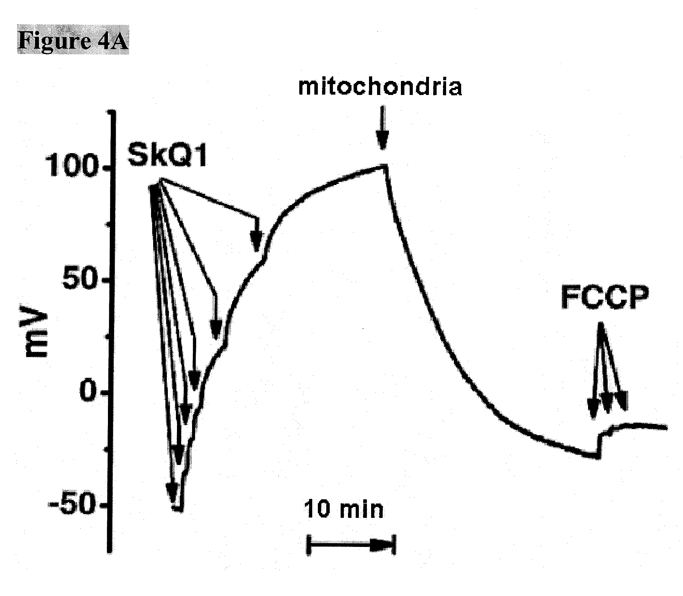

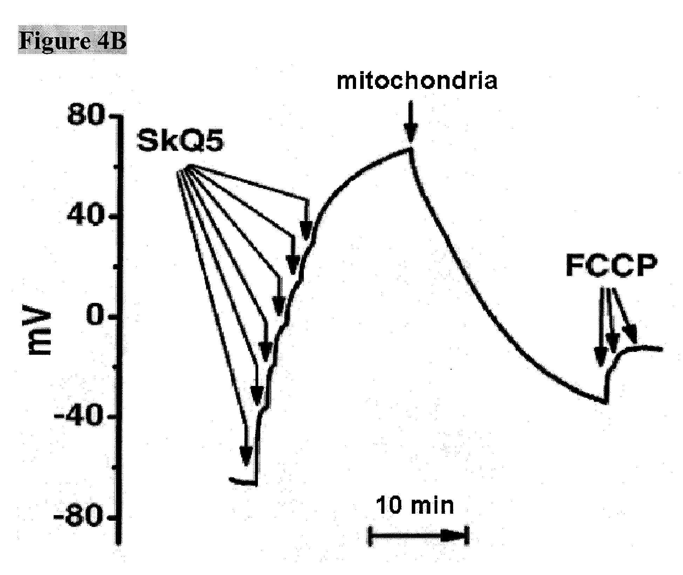

The ability of compounds of general formula (I) to accumulate in mitochondria was tested using a tetraphenylphosphonium-selective electrode. Said method can be used only for compounds of general formula (I) in which the lipophilic cation tetraphenylphosphonium is used as the target group. With this electrode, it is possible to measure distribution of tetraphenylphosphonium cation (or compounds which comprise this cation) between the mitochondrial matrix and the medium. **Figure 4** shows that SkQ1 accumulates in mitochondria within 15-20 min. The fall in mitochondrial membrane potential caused by oxidative phosphorylation uncoupler FCCP leads to release of a relatively small number of SkQ1 from mitochondria. Given that SkQ1 is a lipophilic cation (the octanol/water distribution ratio for SkQ1 is 20000/1), the main amount of SkQ1 accumulates in the mitochondrial membrane independent of the degree of energization of the mitochondria. If less lipophilic SkQ5 is used in place of SkQ1, the level of energy-independent accumulation is markedly reduced. Said method allows to investigate the efficiency of accumulation of compounds of general formula (I) in mitochondria, the dependence of the rate of their accumulation on the functional state of mitochondria, as well as to predict the potential bioavailability of tested compounds.

### ii) Measurement of ability of compounds of general formula (I) to be reduced with mitochondrial respiratory chain.

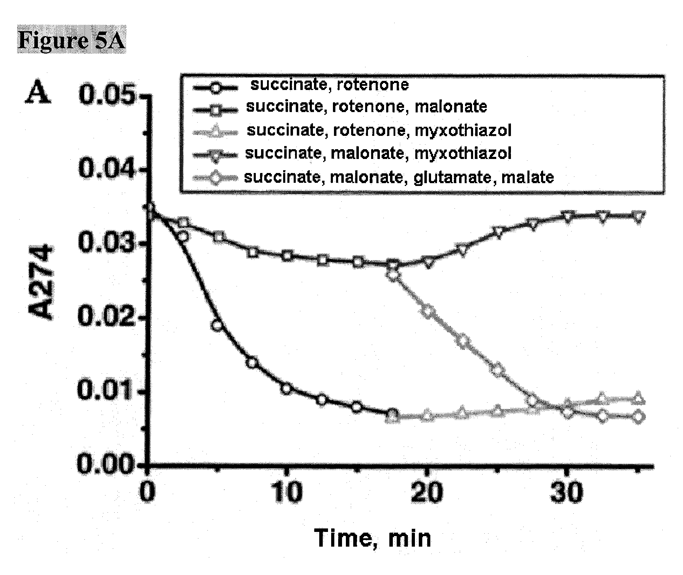

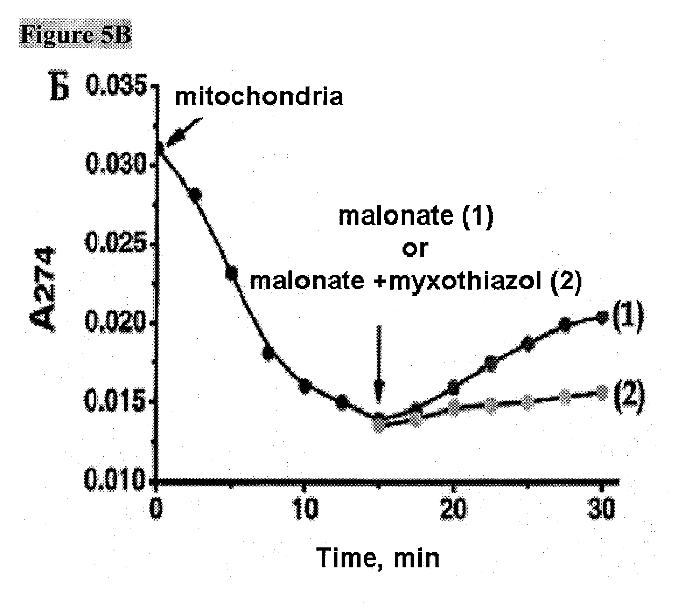

A key advantage of mitochondria-addressed antioxidants proposed in the present invention is their ability to be reduced with mitochondrial respiratory chain. To study a reduction of compounds of general formula (I) with mitochondrial respiratory chain, the rate of change in the ratio between the oxidized and reduced forms of the compounds in the presence of respiratory substrates in isolation medium of rat liver mitochondria was measured. The measurements were performed in the presence of mitochondria. The experiments showed that SkQ1 can be reduced with the mitochondrial respiratory chain. The use of various substrates for oxidation showed that SkQ1 can be reduced with both complex I (mitochondria were energized with glutamate and malate) and complex II (substrate for oxidation was succinate) **(****Figure 5A****)**. To eliminate influence of endogenous substrates, complex I inhibitor rotenone as well as complex II inhibitor malonate were used. Inhibition of complex II by malonate in the presence of rotenone stimulated oxidation of SkQ1, probably by complex III. Inhibition of complex III by myxothiazol, in turn, prevented that oxidation that confirms the ability of complex III to oxidize SkQ1 **(****Figure 5B****)**. Oxidation of SkQ1 with complex III is much slower than its reduction with complex I and complex II **(****Figure 5B****).** These data indicate that in energized mitochondria, SkQ1 is mostly in the reduced state (quinol form) in which it manifests antioxidant properties.

Thus, said method allows to study the ability of compounds of general formula (I) to be reduced with mitochondrial respiratory chain, in addition, on the basis of the data it is possible to predict pro-oxidant or antioxidant properties of test compounds.

### iii) Testing antioxidant activity of compounds of general formula (I) under conditions of mitochondrial oxidative stress induced by a mixture of FeSO₄ and ascorbate.

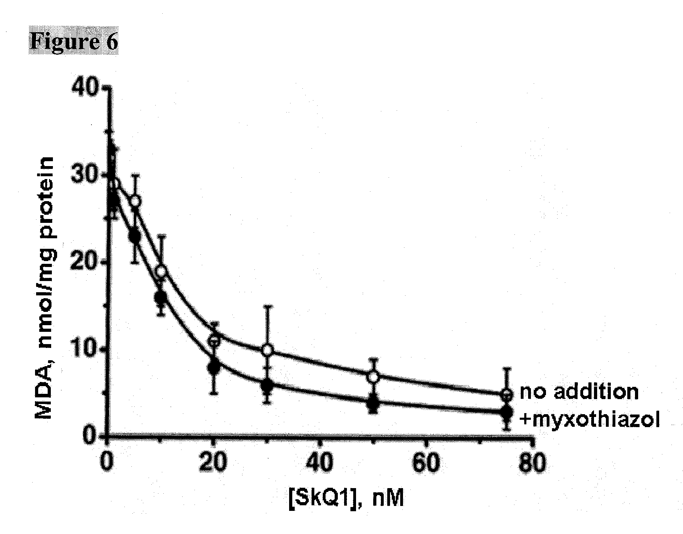

One of the most widely used methods for determination of oxidative stress in mitochondria, cell cultures or tissues is a method for quantitative determination of malondialdehyde. Oxidative stress in these cases can be induced by a variety of ways: by tert-Butyl hydroperoxide, Cumene hydroperoxide, hydrogen peroxide, xanthine/xanthine oxidase, a mixture of ferrous sulfate and potassium ascorbate etc. To initiate oxidative stress in the experiments, a mixture of ferrous sulfate and potassium ascorbate was used. It is known that in mitochondrial metabolism, a certain amount of H₂O₂ is formed which under physiological conditions is not dangerous as quickly utilized by various antioxidant systems. Addition of ferrous sulfate in combination with potassium ascorbate to medium containing mitochondria causes a reaction of ferrous iron with H₂O₂ (the Fenton reaction) to form the highly reactive hydroxyl radical (Fe²⁺ + H₂O₂ → Fe³⁺ + ·OH + ⁻OH). In turn, the hydroxyl radical reacts with unsaturated fatty acids in membranes and stimulates their free radical oxidation, ultimately leading to accumulation of malondialdehyde. Such a model is well suited for studying effectiveness of various antioxidants including compounds of general formula (I). **Figure 6** shows the results of testing antioxidant activity of SkQ1. As can be seen, the highest antioxidant activity of SkQ1 is already manifested at concentrations of 20-50 nM. Addition of myxothiazol (complex III inhibitor) to the mitochondrial suspension prevents oxidation of SkQ 1 with complex III that greatly improves the antioxidant capacity of SkQ1. The results confirm the antioxidant capacity of SkQ1 and, moreover, show the importance of the ability of antioxidants to be reduced with the mitochondrial respiratory chain.

Thus, the method for quantitative measurement of malondialdehyde with a high degree of accuracy allows to predict pro-oxidant or antioxidant properties of test compounds as well as to test their effective concentrations.

### Experimental example 5. Testing antioxidant or pro-oxidant action of mitochondria-addressed compounds in animal, plant, bacterial or yeast cell cultures.

Given the large number of different methods for testing biological activity of compounds in cell culture, this section will describe only those methods which, because of their simplicity and informativity, are the most suitable for initial testing compounds of general formula (I).

### a. Testing action of mitochondria-addressed compounds in animal cell cultures.

Human uterine carcinoma cell line HeLa and normal human diploid fibroblasts derived from lung and skin were selected as a model for testing antioxidant capacity of compounds of general formula (I). Testing antioxidant capacity of the compounds was performed using the methods of cytofluometry and fluorescence microscopy. In preliminary experiments for each cell culture the optimal concentration of H₂O₂ which causes a significant (60-80%) cell apoptosis with no visible signs of necrosis was selected. To determine the chromatin condensation and fragmentation which occurs in apoptotic cells, the fluorescent dye Hoechst was used. The dye at a concentration of 1 µg/ml was added to live or fixed cells at the end of incubation for 30 minutes. To determine necrosis, the fluorescent dye propidium iodide (PI) at a concentration of 2 µg/ml was added to non-fixed cells. The percentage of apoptotic and necrotic cells was determined by counting the number of cells with fragmented nuclei and cells permeable to propidium iodide, respectively.

In experiments with penetrating mitochondria-addressed antioxidants one needs to be aware that depending on penetrating ability of the compounds, the time necessary for their accumulation in mitochondria from cell culture may be different.

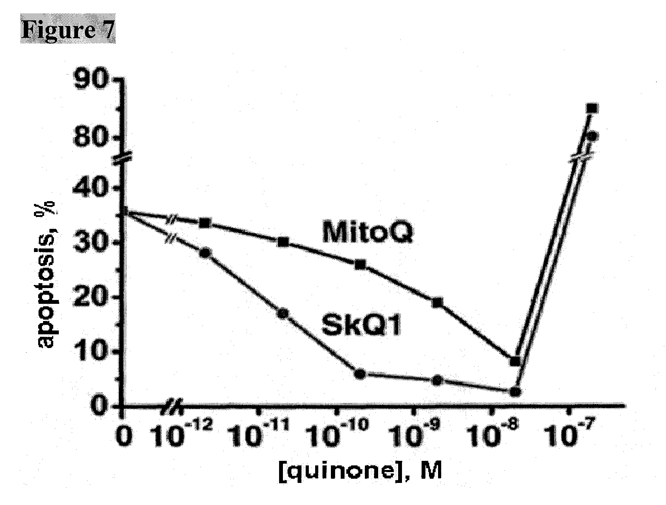

In particular, we showed that SkQ1 and MitoQ increase resistance of cells to H₂O₂ only if the cells are incubated with them for 5-7 days. Oxidative phosphorylation uncoupler FCCP causing the fall in mitochondrial membrane potential prevented the protective effects of SkQ1 and MitoQ. This is an important control that indicates that the tested compounds are indeed mitochondria-addressed. SkQ1 and MitoQ, being mitochondria-addressed antioxidants, exert their effects at very low concentrations. In particular, SkQ1 exerts its protective effect even at a concentration of 0.2 nM **(****Figure 7)**. **Figure 7** shows that SkQ1 protects cells against death much more effectively, as compared with MitoQ, i.e., SkQ 1 is a more effective antioxidant. Like any antioxidants, SkQ1 and MitoQ have limiting concentrations above which they show pro-oxidant activity, in particular, at a concentration of SkQ1 and MitoQ above 0.5 µM they manifest pro-oxidant activity leading to stimulation of cell death induced by H₂O₂.

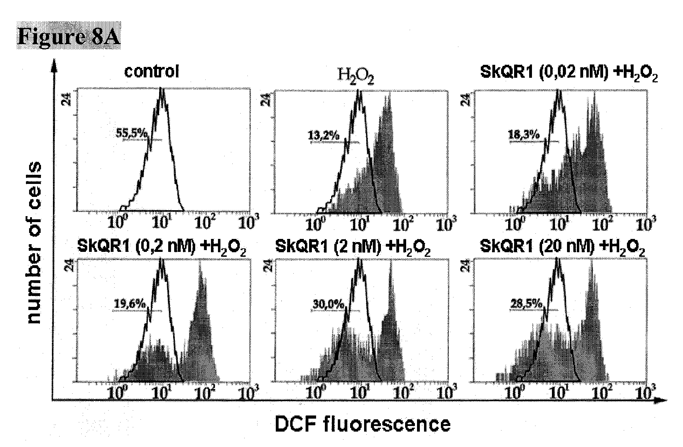

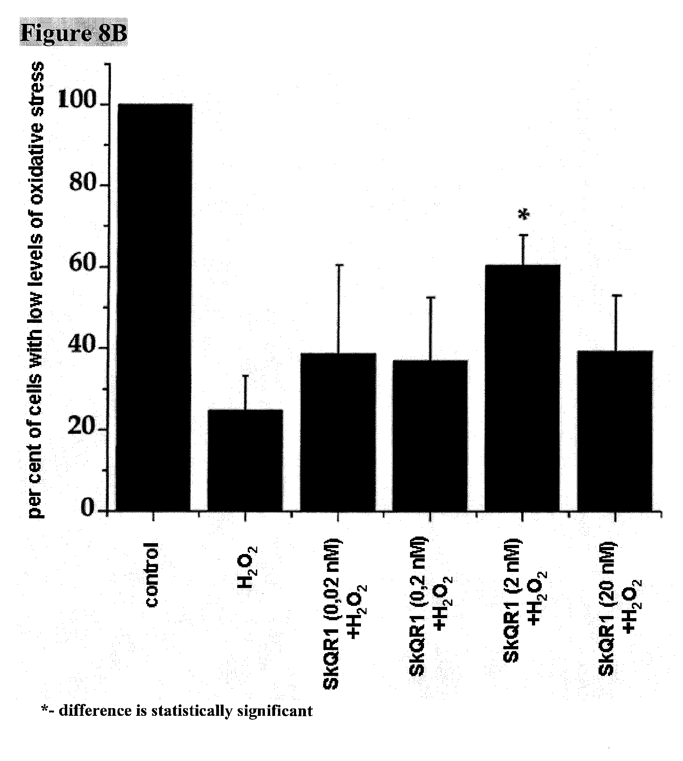

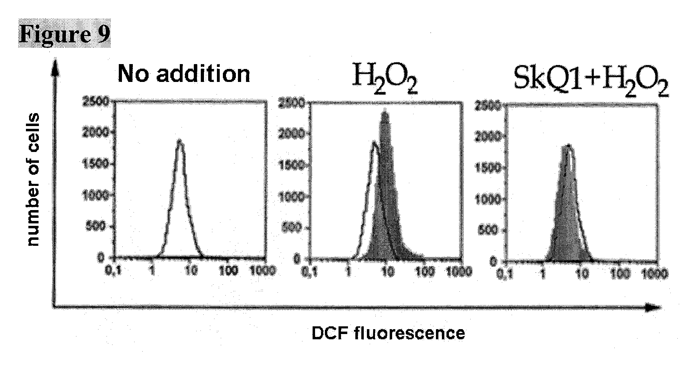

To measure the level of oxidative stress stimulated by H₂O₂, cells were stained with fluorescent dye DCF-DA (2',7'-dichloro-dihydrofluorescein diacetate) and then the level of fluorescence of the dye was measured by cytofluorimeter. With this method, it was shown that incubation of cells with SkQ1 or MitoQ for 1 - 5 hours did not prevent H₂O₂-induced oxidative stress in cells. At the same time, SkQR1 having higher penetrating ability (hydrophobic cation - rhodamine G moiety, was used as the target group in place of tetraphenylphosphonium) already has an antioxidant activity within a given time window, and at lower concentrations as compared with SkQ1 **(****Figure 8A**,**B**). SkQ1 **(****Figure 9****)** as well as MitoQ **(not shown)** exhibited their antioxidant properties only after 5 - 7 days of incubation with the cells. The resulting time dependencies are well correlated with penetrating ability of SkQR1, SkQ1 and MitoQ (penetrating abilities of these cations can be described by the following sequence: SkQR1 > SkQ1> MitoQ).

Thus, said methods allow to determine the ability of compounds of general formula (I) to protect cells against death caused by oxidative stress. In addition, said methods may help in predicting therapeutic doses and timing of administration of preparations based on compounds of general formula (I).

### b. Testing action of mitochondria-addressed compounds in E. coli cells.

To test pro-oxidant and antioxidant properties of mitochondria-addressed compounds of general formula (I), a method for determining oxidative stress in *E*. *coli* cells was developed. For this purpose, biosensor system based on luxAB genes encoding bacterial luciferases to study effect of penetrating ions on oxidative stress in a bacterial cell was created. Luciferases are now widely used in studies on molecular genetics (reporter genes), biochemical assays (e.g., determination of trace amounts of ATP), genetic engineering works (selection), biotechnology and ecology (biosensors) etc. High sensitivity, the ease of detection of light signal with the use of luminometer or scintillation counter, direct proportionality between the amount of the enzyme luciferase and bioluminescence intensity within a few orders of magnitude, a possibility of measuring both *in vitro and in vivo* (without damaging cells) and other benefits support application of luciferase genes in various genetic and biochemical tests. In a method developed, genes encoding luciferase from terrestrial bacteria *Photorhabdus luminescens* were used [1]. Gram-negative bacteria *Ph. luminescens* are symbionts of entomopathogenic nematodes. Luciferase from *Ph. luminescens* is characterized by high thermal stability (it remains active at temperatures up to 45 °C) that facilitates the use of the *lux* genes as reporters.

To test chemical contaminants (toxicants) in water, soil, food, air etc., the *lux*biosensors are currently being used in two variants:
1) based on bioluminescence quenching by toxicant;
2) based on induction (increase) of bioluminescent intensity by toxicant.

Methods related to the first variant include the use of a mechanism of inhibitory effect of toxic substances on cell metabolism, mainly on the respiratory chain, that indirectly affects the luciferase reaction resulting in a decrease in bioluminescent intensity of cell suspension.

Methods related to the second variant are based on induction (increase) of intensity of cells bioluminescence induced by a toxicant. These methods include various options for the use of specific regulatory elements developed by bacteria in the process of evolution and specifically responsive to the presence of a particular chemical substance in the environment. The above mentioned group of biosensors provides both specificity and high sensitivity because they are based on the interaction of a receptor protein (repressor or activator) with a chemical compound. In bacteria, one can distinguish regulatory systems specifically reacting to toxicants which act on: 1) cell membranes, 2) proteins, 3) chromosome (DNA), and 4) inducing oxidative stress in a cell. In addition, bacteria have regulatory systems which specifically react to heavy metals and arsenic ions. The *grp*E: P_{*grp*E} promoter can be used as a biosensor for toxicants which act on cellular proteins (for example, various phenol derivatives, alcohols). Said promoter is located in the bacterial genome upstream of heat shock genes and is activated only when modified, denatured proteins appear in a cell. The P_{recA} SOS promoter is used as a biosensor for DNA-tropic agents (mitomycin C, methyl methanesulfonate, dioxins, as well as ultraviolet and ionizing radiation). The LexA protein is a repressor. The P_{recA} promoter is activated only upon the induction of damage to the genome, i.e., to DNA molecules. To detect substances inducing oxidative stress in a cell (forming the hydroxyl radical (OH·), superoxide ion-radical (O₂⁻), hydrogen peroxide (H₂O₂) in a cell), the P_{katG} and P_{soxS} promoters are used. The P_{katG} promoter (activator OxyR) specifically reacts to hydrogen peroxide, organic peroxides etc. The P_{soxS} promoter is activated when superoxide ion-radical appears in the environment. On the basis of the said inducible promoters the *lux-*biosensors were developed.

All the promoters used in the developed method, with corresponding regulatory regions, were obtained from the genome of the *Escherichia coli* K12 MG1655 bacteria by PCR method with the use of specific synthesized primers. Non-promoter vector with the pBR322 replicon and *bla* gene responsible for resistance to ampicillin (selective marker) was used as a vector. Embedding promoter region into plasmid was carried out at the *Eco*RI-*Bam*HI sites. The *lux* operon of *Ph. luminescens* consisting of five genes, *lux*CDABE, was selected as the lux cassette.

All the biosensors were tested for suitability to work with mitochondria-addressed antioxidants of general formula (I).

It turned out that mitochondria-addressed antioxidants of general formula (I), in particular, SkQ1 and MitoQ, are most likely to have high specificity to biosensors anyhow related to oxidative stress, as their structure comprises quinone derivatives and they accumulate in charged membranes with high efficiency. Therefore, the use of the pLUX::PkatG and pLUX::PsoxS biosensors seems to be optimal. DNA damage fixation in oxidative stress and the effect of penetrating ions on this process is possible when the pLUX::PrecA biosensor is used. The pLUX::PgrpE and pLUX::P1ac biosensors apparently will be used as positive and negative controls.

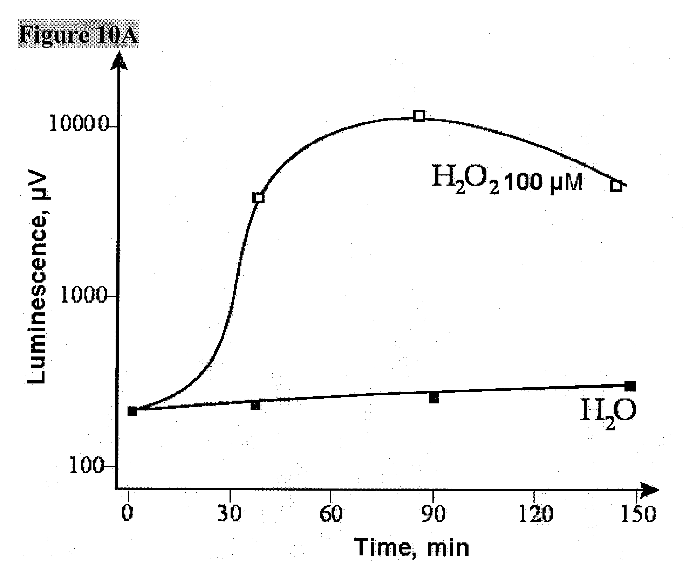

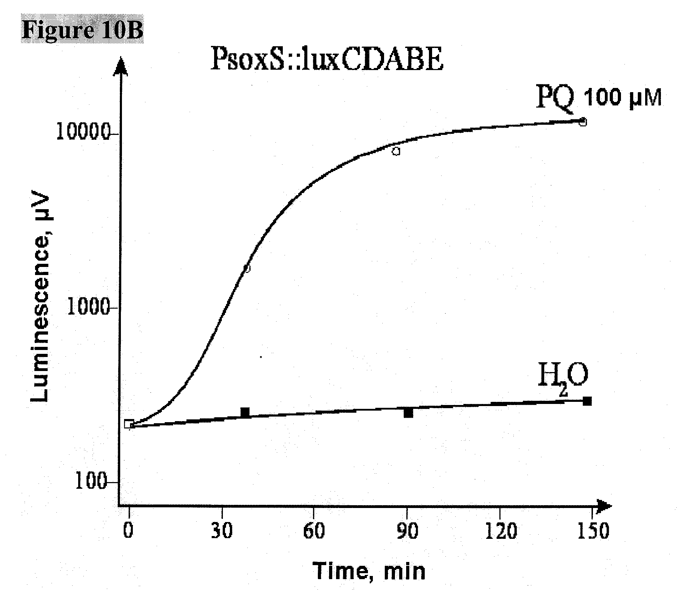

In the first phase of testing, conditions for oxidative stress induction, at which the maximum induction of luminescence occurs, were selected. **Figure 10** shows the ability of H₂O₂ and paraquat to induce bioluminescence of biosensor. Induction of luminescence in *E. coli* MG1655 pLUX::PkatG in the presence of H₂O₂ becomes evident within 15 minutes and reaches a maximum value for one hour **(****Figure 10A****).** Ratio of the intensity of luminescence between control cells and induced cells is 1/80 at optimal concentrations of H₂O₂. Induction of luminescence in *E. coli* MG1655 pLUX::PsoxS in the presence of paraquat becomes evident within 15-20 minutes and reaches a maximum value for two hours **(****Figure 10B****).** Ratio of the intensity of luminescence between control cells and induced cells is 1/100 at optimal concentrations of paraquat.

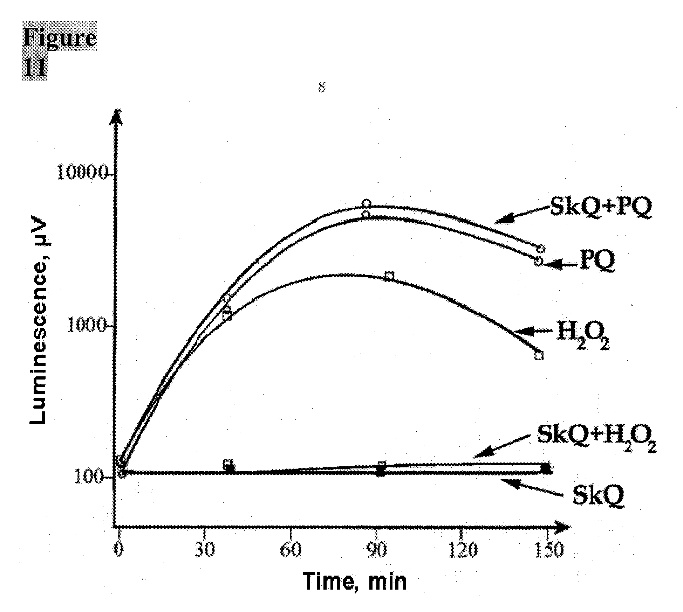

At the next stage, it becomes possible to test pro-oxidant or antioxidant capacities of compounds of general formula (I). With the help of the pLUX::PkatG and pLUX::PsoxS biosensors, antioxidant properties of SkQ under conditions of oxidative stress in *E.coli* were tested. It was shown that 10 µM SkQ efficiently protects a cell against superoxide anion radicals resulting from oxidative stress induced by hydrogen peroxide, wherein said concentration of SkQ had no appreciable effect on oxidative stress induced by paraquat **(****Figure 11****).** This discrepancy may be due to different ways of generation of reactive oxygen species by hydrogen peroxide and paraquat. With hydrogen peroxide, oxidative stress is induced for a short time (H₂O₂ is actively decomposed by antioxidant defense enzymes in a cell), whereas with paraquat, oxidative stress lasts much longer. In addition, it was shown that in the *E. coli* cells, the multidrug resistance (MDR) system that exports positive cations out of a cell actively functions. The activity of these enzymes dramatically reduces efficacy of SkQ1 and its analogs. Thus, with H₂O₂, SkQ1 has time to protect cells against oxidative stress, prior to be exported out of a cell by MDR proteins, whereas with paraquat, SkQ1 is no longer effective.

The results showed that the developed test method is reliable and fast tool for testing pro-oxidant or antioxidant capacity of compounds of general formula (I).

### Experimental example 6. In vivo testing biological activity of compounds of general formula (I) in animals and plants.

*In vivo* testing of compounds of general formula (I) in animals or plants can be applied only to those compounds which have passed all previous tests of the section and have demonstrated potential biological activity. This is due to the fact that for preparation of *in vivo* experiments it is necessary to have complete information about potential targets for drug action that is necessary to develop a model that should be used by a researcher to obtain the most informative results. For *in vivo* experiments at the pre-selection stage, the simplest methods that allow to evaluate the principal possibility of drugs to be biologically active have to be used. For this purpose, small invertebrates such as the crustaceans *Ceriodaphnia affinis* can be used as a model object. Such objects, in particular, zooplankton organisms serve as a popular test object at the estimation of environmental pollution, study on biological effects of extracts of materials, food products, medical preparations. In the overwhelming number of applications, the estimation is made in short-term experiments taking into account survival, behavior of test objects and violations of certain physiological functions. For identification of effects of weak influences in chronic mode, apart from said parameters, such integral individual parameters as growth and reproduction are also under control.

In quantifying effect of chemical agents on test objects in a chronic mode, the phenomenon called the phase character of toxic effect, i.e. alternation of depression and stimulation of activity of a biological function or development of a structural element caused by potentially toxic substances, is manifested. As a result, many potential toxicants at certain concentrations may have a temporary stimulating effect on certain functions and on a test object in its entirety. Thus, an important criterion for a favorable effect of the preparations is an organism's whole life span because in this case the risk of being in a favorable phase of the preparation which is really toxic can be excluded.

The effect of the preparation SkQ1 at different concentrations on basic life functions of the crustaceans *Ceriodaphnia affinis* for the duration of their natural life was studied with particular attention to stimulating effect of the preparation.

In the first series of experiments (the number of animals in each series was 20 individuals) survival of the crustaceans in the presence of ethanol (0.79 mg/l) did not differ from control **(****Figure 4****).** At concentrations of 5.5 and 0.55 nM SkQ1, survival of the crustaceans was higher than in control, whereas at a concentration of 55 nM, survival was lower. Time of death in a fixed population of the crustaceans at concentrations of 0.55 and 5.5 nM SkQ1 increased during the entire observation period, whereas time of death in 50% exceeded the parameter in control 2 and 1.4 times, respectively **(Table 1).**

**Table 1. Fixed time (days) of death of Ceriodaphnia affinis caused by SkQ1**

| **Concentration** | **LT_{25%}** | **LT_{50%}** | **LT_{75%}** |
|---|---|---|---|
| **Control** | 16 | 18 | 29 |
| **Ethanol, 0.79 mg/l** | 14 | 18 | 25 |
| **SkQ1, 0.55 nM** | 22 | 36 | 45 |
| **SkQ1, 5.5 nM** | 18 | 26 | 46 |
| **SkQ1, 55 nM** | 9 | 12 | 17 |

The average life span of the crustaceans at concentrations of 0.55 and 5.5 nM SkQ1 was higher than in control, at a concentration of 55 nM SkQ1 ― lower, these differences were statistically significant **(Table 2).**

**Table 2. Average life span of Ceriodaphnia affinis caused by SkQ1**

| **Concentration** | **M±m** | **t_{d}** | **% of control** |
|---|---|---|---|
| **Control** | 20.2 ± 4.99 | | |
| **Ethanol, 0.79 mg/l** | 19.55 ± 5.26 | 0.18 | 96.78 |
| **SkQ1, 0.55 nM** | 33.55 ± 6.21 | *3.28** | 166.09 |
| **SkQ1, 5.5 nM** | 31.25 ± 8.11 | *2.27** | 154.7 |
| **SkQ1, 55 nM** | 12.85 ± 2.79 | *2.52** | 63.61 |

| | | | |
|---|---|---|---|
| *- difference is statistically significant | | | |

Thus, the results indicate the ability of SkQ1 to have beneficial effects on the life activity of small invertebrates resulting in increasing their life span. In addition, concentrations of compound that has a beneficial effect on an organism were selected, that can be used in experiments to test biological activity of test preparations of general formula (I) in higher animals.

### Experimental example 7. Method for purification of mitochondria-addressed antioxidant using 'molecular trap'

1. Initial technical product PDTP (5 g) after silica gel pretreatment in ethanol-chloroform (1:9) system has a purity of about 85%. The content of the reduced form is 8%.
   To remove basic impurities, HPLC method was used. C₁₈ column, 500 × 45 mm. Mobile phase - salt-free unbuffered water-ethanol solution. Gradient mode. System A - 15% ethanol, system B - 40% ethanol.
   After collection of central fractions a purity of the preparation is about 92%. The content of the reduced form is 6%.
   Comparative qualitative reaction of initial and purified product to bromides shows retention of bromide ion after chromatographic purification.
   Analytical HPLC on a C₁₈ column, 250 × 4.6 mm in a system of 0.05% trifluoroacetic acid in 65% acetonitrile in water also shows approximately the same peak intensity of the bromide ion (at the beginning of the chromatogram) for initial and purified product.
2. After purification by HPLC (3.8 g), solvent evaporation and drying under a high vacuum, the product has a form of thick, clear oil, dark brown in color. To minimize the content of the reduced form in the preparation, a variant of the method of 'molecular trap' was used.

A flask of oil is poured with 200 ml of hexane, then 5 ml of acetone is added and the mixture is stirred vigorously with a magnetic stirrer for 30 min. Then the solvent layer is carefully decanted off. A chromatographic control of the decanted portion of the solvent and the remaining oil is made. HPLC chromatogram of solvent contains a small peak of acetone and only an intense peak of the reduced form; the main oxidized form is missing.

The chromatogram of the desired product shows clearly decrease in the content of the reduced form.

To further minimize the content of the reduced form, repeating the procedure several times may be required.

It is possible to carry out the procedure in automatic mode with continuous feed of fresh portion of solvent and disposal of used solution.

It is important to note that with said method, there is no loss of basic substance. Bromide ion is preserved.

Purity of the product is about 97%. The content of the reduced form is not more than 1.0%.

### 2. Gel chromatography in ethanol solution.

This purification method is suitable as a final stage prior to dosage bottling of concentrated solution of the preparation, drying and putting in storage.

Approximately 3.7 g of the preparation is dissolved in 5-6 ml of ethanol and subjected to chromatography on a 600 × 10 mm column of Sephadex LH-20 preequilibrated with absolute ethanol (Spectrophotometric Grade).

Head and tail fractions are discarded. Purity of the main faction is at least 98%. The content of the reduced form is 0.8-0.9%. The concentration of the preparation can reach 150-200 mg/ml. This solution is convenient for preparation of aliquots and drying of the substance in the final form.

### Experimental example 8. Synthesis of plastoquinonyl-decyl-triphenylphosphonium (PDTP) bromide

The synthesis involves the following steps:
1. Oxidation of 2,3-dimethylphenol (1) to 2,3-dimethyl-1,4-benzoquinone (2) with the Jones reagent.
2. Attachment of 11-bromo-undecanoic acid (3) to triphenylphosphine with the formation of (10-carboxy-decyl)triphenylphosphine bromide (4).
3. Formation of the desired compound (5) by the reaction of the produced compound (4) with 2,3-dimethyl-1,4-benzoquinone (2) in the presence of silver nitrate and ammonium persulfate.

The scheme of the synthesis is shown in **Figure 2****.**

### Synthesis of 2,3-dimethyl-1,4-benzoquinone (2)

The Jones reagent (solution of 110 g (0.37 mol) of Na₂Cr₂O₇ × 2H₂O in a mixture of 157 ml of water and 70 ml of concentrated sulfuric acid) was added with stirring to a solution of 20 g (0.16 mol) of 2,3 dimethylphenol in 230 ml of ether and the mixture was stirred for 24 hours. The mixture was extracted with ether, the combined ether extracts were washed and then dried with calcined magnesium sulfate and after removal of the solvent on a rotary evaporator the residue was subjected to flash chromatography on silica gel in chloroform. The yield of compound **2** in a form of yellow crystalline substance is 8.7 g (40%).

TCX: R_{f} (CHCl₃) = 0.46; HPLC: τ = 17.4 min (0-90 % B for 26.4 min; A: 10 mM H₃PO₄, B: AcCN), m.p.58 °C (56.5-57.5 °C)¹; UV (CH₃OH): λmax 209 nm, 256 nm, 344 nm; ESI MS: m/z calculated for C₈H₈O₂ 136.15; found 136.2.

### Synthesis of (10-carboxy-decyl)triphenylphosphine bromide (4)

588 mg (2.24 mmol) of triphenylphosphine was added to 530 mg (2 mmol) of 11-bromo-undecanoic acid and the mixture was kept in a sealed tube at 85 °C for 12 hours. Then the mixture was subjected to separation on a silica gel column in a system of chloroform - methanol (9: 1). The yield of compound **4** in a form of clear oil is 895 mg (85%).

TCX: R_{f} 0.52 (chloroform - methanol, 4:1); HPLC: τ = 7.28 min (5 ― 95% B for 11.5 min; A: 0.1% TFA; B: 0.1% TFA in acetonitrile); UV spectrum (0.1% TFA ― acetonitrile, 38:62): λₘₐₓ 200 nm, 224 nm, 268 nm; ESI MS: calculated for C₂₉H₃₆OP: 447.6; found m/z 448.2 (MH⁺; 100%).

### Synthesis of [10-(4,5-dimethyl-3,6-dioxo-cyclohexa-1,4-dien-1-yl)decyl]-triphenylphosphonium bromide, PDTP bromide (5)

Solution of 228 mg (1 mmol) of ammonium persulfate in 5 ml of water was added to solution of 135 mg (1 mmol) 2, 526 mg (1 mmol) 4 and 85 mg (0.5 mmol) of silver nitrate in 40 ml of a mixture of acetonitrile and water (1:1) at 80-90 °C. The mixture was heated with stirring at the same temperature for 12 hours.The mixture was diluted with water and extracted with dichloromethane. After evaporation of dichloromethane to a small volume, the product was precipitated with diethyl ether. The solution was decanted from the precipitate, the precipitate was reprecipitated several times. At the end, the precipitate was purified on a silica gel column in a mixture of dichloromethane - ethanol (in a ratio of 9:1). The yield of [10-(4,5-dimethyl-3,6-dioxo-cyclohexa-1,4-dien-1-yl)decyl]-triphenylphosphonium bromide (PDTP bromide) is 35%.

TCX: R_{f} (CHCl3 ― CH3OH, 4:1) = 0.66; HPLC: τ = 10.1 min (5-95 % B for 12 min; A: 0.05% TFA, B: 0.05% TFA in AcCN); UV (CH3OH): λmax 198 nm, 226 nm, 260 nm (ε₂₆₀= 18652 cm-1*M-1), 352 nm; ESI MS: m/z calculated for C₃₆H₄₂O₂P 537.69; found 537.3.

### Experimental example 9. Synthesis of mitochondria-addressed compounds ― promising mitochondria-addressed antioxidants.

Structures of berberine and palmatine SkQ derivatives:

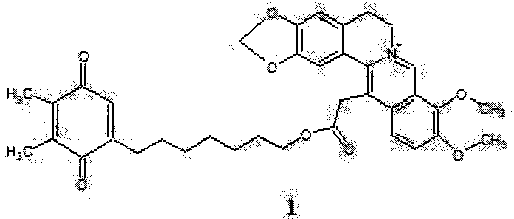

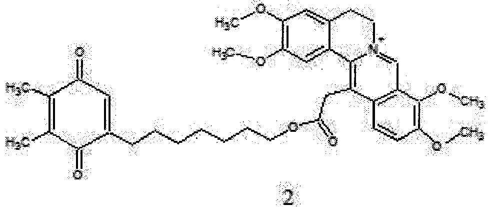

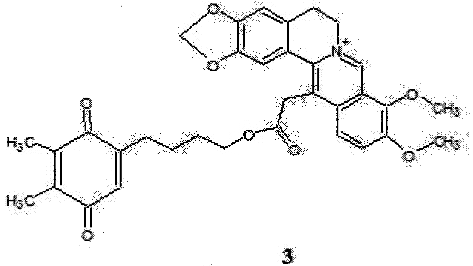

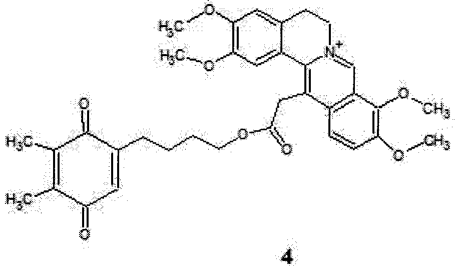

Scheme of synthesis of 9,10-dimethoxy-13-[7-(4,5-dimethyl-3,6-dioxocyclohexa-1,4-dien-1-yl)heptyloxycarbonyl-methyl]-5,6-dihydrobenzo[*g*]-1,3-benzodioxole[5,6-*a*]quinolizinium bromide, 1 (13-[7-(4,5-dimethyl-3,6-dioxo-cyclohexa-1,4-dien-1-yl)heptyloxycarbonyl-methyl]berberine bromide)

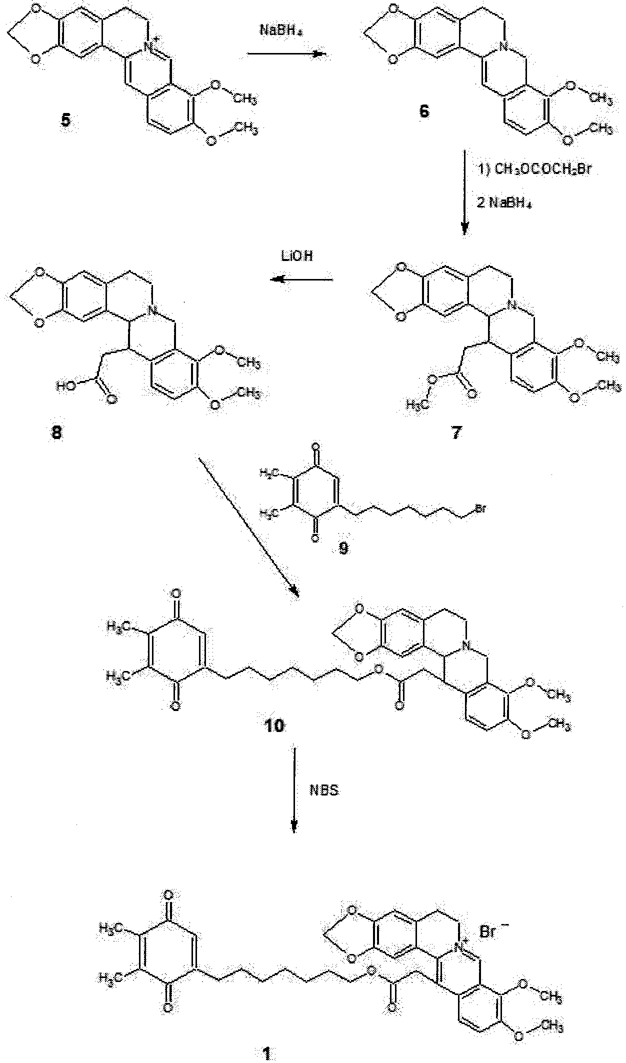

Compound **1** was produced on the basis of berberine bisulfate (**5**) which was reduced with sodium borohydride in pyridine for 30 min at room temperature and after crystallization from water compound **6** was produced with 91 % yield. Compound **6** was alkylated with bromoacetic acid methyl ester (1 hour, 100 °C) followed by reduction of intermediate compound with sodium borohydride (30 min, room temperature) to give compound **7** which was isolated by extraction with ether from an aqueous solution (80% yield) and saponified by 1% water-methanol solution of lithium hydroxide by boiling for 1.5 hours to give compound **8.** After crystallization from water the yield of compound **8** appeared to be 61 %. Compound **8** was converted into cesium salt which was condensed with previously synthesized derivative of 2,3-dimethyl-1,4-benzoquinone **9** at 60 °C for 48 hours. Compound **10** was oxidized with N-bromosuccinimide (NBS) in methylene chloride solution for 1 hour, following removal of excess NBS by washing the organic phase with water and its drying, the mixture was evaporated and the final compound **1** was precipitated with ether. Purification of compound **1** was performed by HPLC (C18) in a gradient of acetonitrile containing 0.05% TFA, in 0.05% aqueous TFA from 30 to 80%. After the last two stages, the overall yield appeared to be 50%.
Compounds **2 ― 4** were similarly produced.

### Characteristics of the compounds produced.

13-[7-(4,5-dimethyl-3,6-dioxo-cyclohexa-1,4-dien-1-yl)heptyloxycarbonylmethyl]berberine: TCX: R_{f} (chloroform - methanol, 65:10) = 0.16; R_{f} (chloroform - methanol, 4:1) = 0.39. HPLC: τ = 8.98 min (5-95% B for 11 min; A: 0.05% TFA, B: 0.05% TFA in MeCN; Luna C18(2)' 0.46 × 15 cm, 5 µm, 1 ml/min). UV (ethanol): λmax 262 nm, 350 nm (ε₃₅₀= 23850 cm⁻¹*M⁻¹), 430 nm (ε₄₃₀ 5278 cm⁻¹*M⁻¹). ESI MS: m/z calculated for C₃₇H₄₀NO₈ 626.72; found 626.69.

13-[7-(4,5-dimethyl-3,6-dioxo-cyclohexa-1,4-dien-1-yl)heptyloxycarbonylmethyl]palmatine: TCX: R_{f} (chloroform - methanol, 65:10) = 0.16; R_{f} (chloroform - methanol, 4:1) = 0.39. UV (ethanol): λmax 262 nm, 350 nm, 430 nm. ESI MS: m/z calculated for C₃₈H₄₄NO₈ 642.76; found 642.29.

13-[4-(4,5-dimethyl-3,6-dioxo-cyclohexa-1,4-dien-1-yl)butyloxycarbonylmethyl]berberine: TCX: R_{f} (chloroform - methanol, 65:10) = 0.23; R_{f} (chloroform - methanol, 4:1) = 0.39. HPLC: τ = 7.71 min (5-95% B for 11 min; A: 0.05% TFA, B: 0.05% TFA in MeCN; Luna C18(2)' 0.46 × 15 cm, 5 µm, 1 ml/min). UV (ethanol): λmax 262 nm, 350 nm, 430 nm. ESI MS: m/z calculated for C₃₄H3₄NO₈ 584.64; found 584.22.

13-[4-(4,5-dimethyl-3,6-dioxo-cyclohexa-1,4-dien-1-yl)butyloxycarbonyl-methyl] palmatine: TCX: R_{f} (chloroform - methanol, 65:10) = 0.23; R_{f} (chloroform - methanol, 4:1) = 0.39. HPLC: τ = 7.73 min (5-95% B for 11 min; A: 0.05% TFA, B: 0.05% TFA in MeCN; Luna C 18(2)' 0.46 × 15 cm, 5 µm, 1 ml/min). UV (ethanol): λmax 262 nm, 350 nm, 430 nm. ESI MS: m/z calculated for C₃₅H₃₈NO₈ 600.68; found 600.87.

## Claims

1. Procedure for selection of mitochondria-addressed compounds, antioxidants or pro-oxidants, of general formula (I) 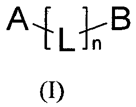
wherein **A** is effector moiety; **L** ― linker group, n - integer from 1 to 20; **B** ― targeting group that provides targeted delivery of the compound into mitochondria, and wherein
**A** may be an antioxidant of general formula (II) 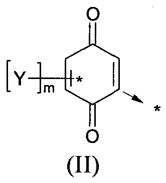 and/or reduced form thereof
wherein **m** - integer from 1 to 3; **Y** - identical or different substituents selected from lower alkyl or lower alkoxy; or two vicinal Y are connected to each other so that they form a structure (III): 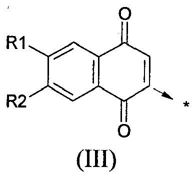 and/or reduced form thereof
wherein R₁ and R₂ are identical or different substituents selected from lower alkyl or lower alkoxy;
**L** ― linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** ― targeting group comprising:
a) either Skulachev-ion Sk:
Sk⁺ Z⁻
wherein Sk ― lipophilic cation;
Z - pharmaceutically acceptable anion;
b) or amphiphilic zwitterion which is able to penetrate into mitochondria in its cationic form;
Sk⁺ as a component of **B** may also be a lipophilic metal-organic compound, in particular, lipophilic metalloporphyrin preferably having a structure: 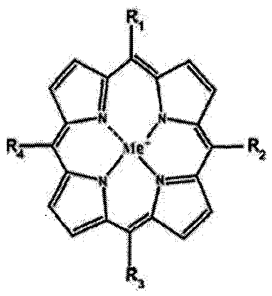 included in the composition of a compound of formula (I) through the moieties designated R₁, R₂, R₃ or R₄; the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn,
comprising the following steps:
a) design of structure of compound based on preliminary forecast of properties of mitochondria-addressed antioxidants.
b) synthesis of compound that corresponds to said structural formula or isolation and purification of compound that corresponds to said structural formula provided that it or its close analogue exists in nature.
c) testing biological activity of compound using a set of tests comprising one and/*or* more tests selected from:
i) testing redox properties and stability of the compounds of general formula (I) *in vitro;*
ii) testing penetrating ability of mitochondria-addressed compounds on artificial black membranes;
iii) testing protective or damaging effect of mitochondria-addressed compounds on membrane lipids and proteins in cell-free model system;
iv) testing antioxidant or pro-oxidant effect of mitochondria-addressed compounds on isolated mitochondria;
v) testing antioxidant or pro-oxidant action of mitochondria-addressed compounds in animal, plant, bacterial or fungal cell cultures;
vi) testing anti-apoptotic or anti-necrotic or pro-apoptotic or pro-necrotic or antioxidant, or pro-oxidant action of mitochondria-addressed compounds in cell cultures;
vii)testing accumulation ofmitochondria-addressed compounds in cells;
viii) testing mitochondria-addressed compounds for their ability to activate or inhibit desired metabolic pathways that is manifested in activation of genes at the transcriptional level, mRNA stability or translation, at the level of protein modifications comprising, in particular, phosphorylation or dephosphorylation, proteolysis, glycosylation, carbonylation and other changes in the activity of proteins or protein complexes, activation or inhibition of metabolic pathways may also be manifested in change of other physiological parameters of a cell including: change in the respiratory rate, change in the rate of production of metabolites, change in the rate of consumption of substrates, change in membrane potential on the outer membrane, on the mitochondrial membrane or membranes of other organelles, change in the ionic conductivity of one or more of said membranes, change in concentrations of ions including change in pH in cellular cytoplasm or in other cellular compartments, change in intracellular transport of biomolecules, vesicles and organelles, change during the cell cycle, change that leads to cell division, cell transformation, cell death or, conversely, to cell survival;
ix) testing biological activity of compounds of general formula (I) *in vivo* in animals or plants including testing compounds in disease models.
d) interpretation of results of testing compounds and selection of the most promising compounds corresponding to parameters required.
e) selection of desired mitochondria-addressed antioxidants or pro-oxidants.

2. Combinatorial library that is a collection of more than one mitochondria-addressed compound corresponding to general formula (I). 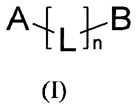 wherein **A** is effector moiety; **L** ― linker group, **n** - integer from 1 to 20; **B** ― targeting group that provides targeted delivery of the compound into mitochondria, and wherein
**A** may be an antioxidant of general formula (II) 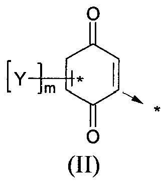 and/or reduced form thereof
wherein **m** - integer from 1 to 3; **Y** - identical or different substituents selected from lower alkyl or lower alkoxy; or two vicinal Y are connected to each other so that they form a structure (III): 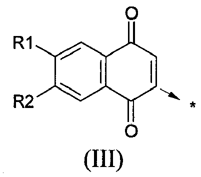 and/or reduced form thereof
wherein R₁ and R₂ are identical or different substituents selected from lower alkyl or lower alkoxy;
**L** ― linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** ― targeting group comprising:
a) either Skulachev-ion Sk:
Sk⁺ Z⁻
wherein Sk ― lipophilic cation;
Z - pharmaceutically acceptable anion;
b) or amphiphilic zwitterion which is able to penetrate into mitochondria in its cationic form;
Sk⁺ as a component of **B** may also be a lipophilic metal-organic compound, in particular, lipophilic metalloporphyrin preferably having a structure: 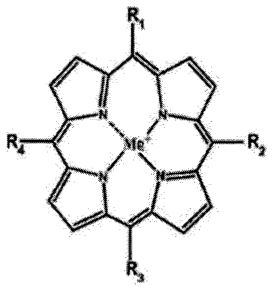
included in the composition of a compound of formula (I) through the moieties designated R₁, R₂, R₃ or R₄; the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn.

3. Procedure according to claim 1 for analysis of combinatorial library of compounds claimed in claim 2.

4. Mitochondria-addressed antioxidants, selected or designed with the use of procedure claimed in claims 1 or 3, with a given ratio between pro-oxidant and antioxidant properties, and described by general formula (I) 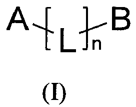 wherein **A** is effector moiety; **L** ― linker group, **n** - integer from 1 to 20; **B** ― targeting group that provides targeted delivery of the compound into mitochondria, and wherein_**A** may be an antioxidant of general formula (II) 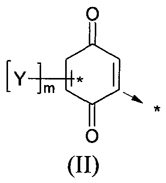 and/or reduced form thereof
wherein **m** - integer from 1 to 3; **Y** - identical or different substituents selected from lower alkyl or lower alkoxy; or two vicinal Y are connected to each other so that they form a structure (III): 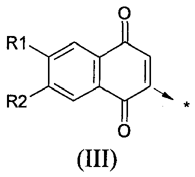 and/or reduced form thereof
wherein R₁ and R₂ are identical or different substituents selected from lower alkyl or lower alkoxy;
**L** ― linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** ― targeting group comprising:
a) either Skulachev-ion Sk:
Sk⁺ Z⁻
wherein Sk ― lipophilic cation;
Z - pharmaceutically acceptable anion;
b) or amphiphilic zwitterion which is able to penetrate into mitochondria in its cationic form
Sk⁺ as a component of **B** may also be a lipophilic metal-organic compound, in particular, lipophilic metalloporphyrin preferably having a structure: 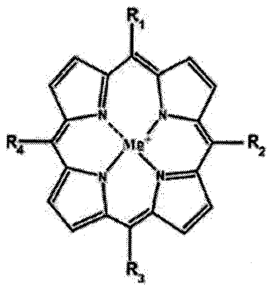 included in the composition of a compound of formula (I) through the moieties designated R₁, R₂, R₃ or R₄; the remaining substituents R₁, R₂, R₃ or R₄ may be selected in accordance with required properties of the compound, for example, in particular, in order to increase or decrease hydrophobicity of the molecule; Me⁺ denotes a metal ion preferably selected from Mn, Fe, Co, Cu, Mg or Zn,
including SkQB1, SkQB1A, SkQB1B, SkQBP1, SkQB5, SkQBP5 and pharmaceutically acceptable salts thereof.

5. Mitochondria-addressed pro-oxidants, selected or designed with the use of procedure claimed in claims 1 or 3, with a given ratio between pro-oxidant and antioxidant properties, and described by general formula (I) 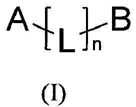 wherein **A** is effector moiety; **L** ― linker group, **n** - integer from 1 to 20; **B** ― targeting group that provides targeted delivery of the compound into mitochondria, and wherein **A** may be a pro-oxidant of general formula (II) 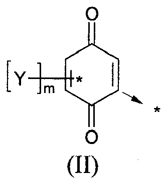 and/or reduced form thereof
wherein **m** - integer from 1 to 3; **Y** - identical or different substituents selected from lower alkyl or lower alkoxy; or two vicinal Y are connected to each other so that they form a structure (III): 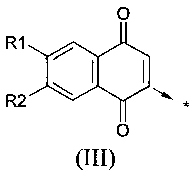 and/or reduced form thereof
wherein R₁ and R₂ are identical or different substituents selected from lower alkyl or lower alkoxy, and wherein **A** is preferably selected from desmethoxyubiquinone or ionol having the structures: 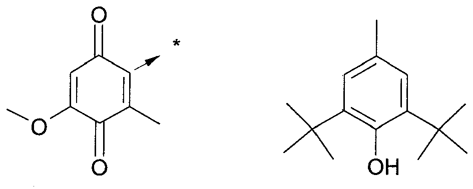 respectively,
**L** ― linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** ― targeting group comprising:
a) either Skulachev-ion Sk:
Sk⁺ Z⁻
wherein Sk ― lipophilic cation;
Z - pharmaceutically acceptable anion;
b) or amphiphilic zwitterion which is able to penetrate into mitochondria in its cationic form, Sk⁺ as a component of **B** may also be a lipophilic metal-organic compound, in particular, lipophilic metalloporphyrin preferably having a structure: 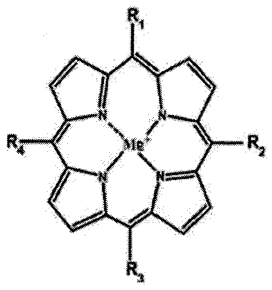 included in the composition of a compound of formula (I) through the moieties designated R₁, R₂, R₃ or R₄; the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn.

6. Procedure for production of mitochondria-addressed antioxidants of general formula (I) 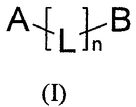 wherein **A** is effector moiety; **L** ― linker group, **n** - integer from 1 to 20; **B** ― targeting group that provides targeted delivery of the compound into mitochondria, and wherein **A** may be an antioxidant of general formula (II) 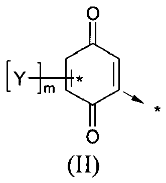 and/or reduced form thereof
wherein **m** - integer from 1 to 3; **Y** - identical or different substituents selected from lower alkyl or lower alkoxy; or two vicinal Y are connected to each other so that they form a structure (III): 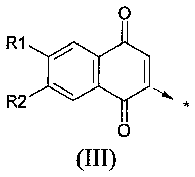 and/or reduced form thereof wherein R₁ and R₂ are identical or different substituents selected from lower alkyl or lower alkoxy;
**L** ― linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** ― targeting group comprising:
a) either Skulachev-ion Sk:
Sk⁺ Z⁻
wherein Sk ― lipophilic cation;
Z - pharmaceutically acceptable anion;
b) or amphiphilic zwitterion which is able to penetrate into mitochondria in its cationic form; Sk⁺ as a component of **B** may also be a lipophilic metal-organic compound, in particular, lipophilic metalloporphyrin preferably having a structure: 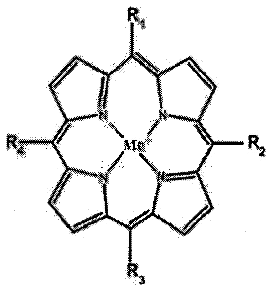
included in the composition of a compound of formula (I) through the moieties designated R₁, R₂, R₃ or R₄, the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn,
comprising synthesis of mitochondria-addressed antioxidant provided that this process comprises at least one of the following stages:
a) production of 2,3-dimethyl-1,4-benzoquinone from available components, in particular, from 2,3-dimethylphenol;
b) production of a lipophilic cation connected to a linker group, preferably production of carboxyalkyl derivative of a lipophilic cation, particularly preferable 10-carboxydecyl triphenylphosphonium.

7. Procedure for purification of mitochondria-addressed antioxidant of general formula (I) 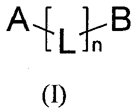 wherein **A** is effector moiety; **L** ― linker group, **n** - integer from 1 to 20; **B** ― targeting group that provides targeted delivery of the compound into mitochondria, and wherein **A** may be an antioxidant of general formula (II) 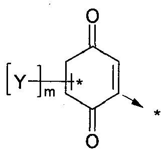 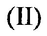 and/or reduced form thereof,
wherein **m** - integer from 1 to 3; **Y** - identical or different substituents selected from lower alkyl or lower alkoxy; or two vicinal Y are connected to each other so that they form a structure (III): 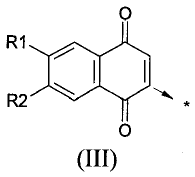 and/or reduced form thereof
wherein R₁ and R₂ are identical or different substituents selected from lower alkyl or lower alkoxy;
**L** ― linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** ― targeting group comprising:
a) either Skulachev-ion Sk:
Sk⁺ Z⁻
wherein Sk ― lipophilic cation;
Z - pharmaceutically acceptable anion;
b) or amphiphilic zwitterion which is able to penetrate into mitochondria in its cationic form; Sk⁺ as a component of **B** may also be a lipophilic metal-organic compound, for example, lipophilic metalloporphyrin preferably having a structure: 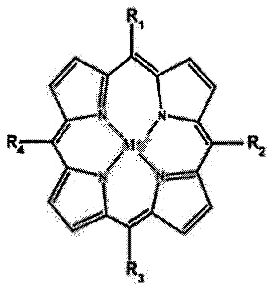
included in the composition of a compound of formula (I) through the moieties designated R₁, R₂, R₃ or R₄, the remaining substituents R₁, R₂, R₃ or R₄ may be selected in accordance with required properties of the compound, in particular, in order to increase or decrease hydrophobicity of the molecule; Me⁺ denotes a metal ion preferably selected from Mn, Fe, Co, Cu, Mg or Zn;
comprising at least one of the following stages:
a) purification using gradient reversed-phase chromatography in salt-free unbuffered mobile phase system,
b) purification using gel filtration chromatography in ethanol,
c) removal of the oxidized form of mitochondria-addressed antioxidant,
d) removal of the reduced form of mitochondria-addressed antioxidant preferably using 'molecular trap' of the reduced form of mitochondria-addressed antioxidant of said general formula, wherein 'molecular trap' is defined as a compound with keto- or quinone-like moiety having oxidative induction potential preferably not less than 0.2 V at neutral pH and preferably is readily soluble in an inert solvent preferably in heptane or hexane.

8. 'Molecular trap' of the reduced form of mitochondria-addressed antioxidant which is a compound with keto- or quinone-like moiety having oxidative induction potential preferably not less than 0.2 V at neutral pH and preferably is readily soluble in an inert solvent preferably in heptane or hexane, and allowing to remove the reduced form from synthesized mitochondria-addressed antioxidant.

9. Procedure for production of mitochondria-addressed antioxidants of general formula (I) 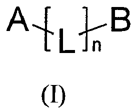 wherein **A** is effector moiety; **L** - linker group, **n** - integer from 1 to 20; **B** - targeting group that provides targeted delivery of the compound into mitochondria; **A** may be an antioxidant of general formula (II) 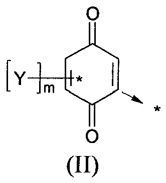 and/or reduced form thereof,
wherein **m** - integer from 1 to 3; **Y** - identical or different substituents selected from lower alkyl or lower alkoxy; or two vicinal Y are connected to each other so that they form a structure (III): 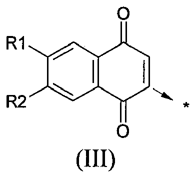 and/or reduced form thereof
wherein R₁ and R₂ are identical or different substituents selected from lower alkyl or lower alkoxy;
**L** - linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** - targeting group comprising:
a) either Skulachev-ion Sk:
Sk⁺ Z⁻
wherein Sk - lipophilic cation;
Z - pharmaceutically acceptable anion;
b) or amphiphilic zwitterion which is able to penetrate into mitochondria in its cationic form; Sk⁺ as a component of **B** may also be a lipophilic metal-organic compound, in particular, lipophilic metalloporphyrin preferably having a structure: 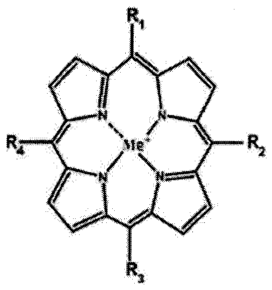
included in the composition of a compound of formula (I) through the moieties designated R₁, R₂, R₃ or R₄, the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn;
in a form of a pharmaceutical drug substance comprising one of the procedures claimed in claims 6 and 7.

10. Pharmaceutical substance based on mitochondria-addressed antioxidant of general formula (I) 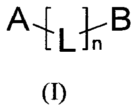 wherein A is effector moiety; **L** - linker group, **n** - integer from 1 to 20; **B** - targeting group that provides targeted delivery of the compound into mitochondria; and wherein **A** may be an antioxidant of general formula (II) 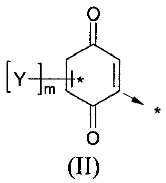 and/or reduced form thereof,
wherein **m** - integer from 1 to 3; **Y** - identical or different substituents selected from lower alkyl or lower alkoxy; or two vicinal Y are connected to each other so that they form a structure (III): 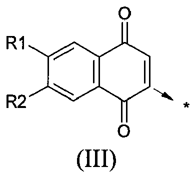 and/or reduced form thereof
wherein R₁ and R₂ are identical or different substituents selected from lower alkyl or lower alkoxy;
**L** - linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** - targeting group comprising:
a) either Skulachev-ion Sk:
Sk⁺ Z⁻
wherein Sk - lipophilic cation;
Z - pharmaceutically acceptable anion;
b) or amphiphilic zwitterion which is able to penetrate into mitochondria in its cationic form; Sk⁺ as a component of **B** may also be a lipophilic metal-organic compound, in particular, lipophilic metalloporphyrin preferably having a structure: 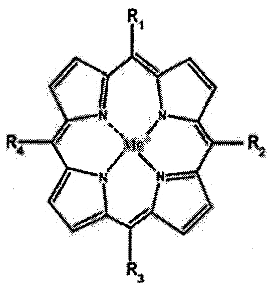 included in the composition of a compound of formula (I) through the moieties designated R₁, R₂, R₃ or R₄, the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn;
in which the ratio between the oxidized and reduced forms is in the range from 0.2% to 99.8% of the oxidized form and in which the ratio between the oxidized and reduced forms of mitochondria-addressed antioxidant is maintained throughout the shelf-life, subject to specified conditions of storage.

11. Pharmaceutical substance claimed in claim 10 in which the ratio between the oxidized and reduced forms is more preferably in the range from 50% to 99.5% of the oxidized form.

12. Pharmaceutical substance claimed in claim 12 in which the ratio between the oxidized and reduced forms is more preferably in the range from 97.5% to 99.5% of the oxidized form.

13. Pharmaceutical substance claimed in claims 10-12 in which the ratio between the oxidized and reduced forms of mitochondria-addressed antioxidant persists for at least 1 month.

14. Pharmaceutical substance claimed in claims 10-12 in which the ratio between the oxidized and reduced forms of mitochondria-addressed antioxidant persists for at least 6 months.

15. Pharmaceutical substance claimed in claims 10-12 in which the ratio between the oxidized and reduced forms of mitochondria-addressed antioxidant persists for at least 2 years.

16. Pharmaceutical substance which is a compound of general formula (I) 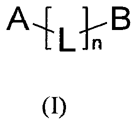 wherein **A** is effector moiety; **L** - linker group, **n** - integer from 1 to 20; **B** - targeting group that provides targeted delivery of the compound into mitochondria; and wherein **A** may be an antioxidant of general formula (II) 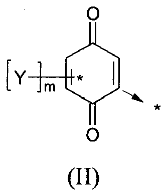 and/or reduced form thereof,
wherein **m** - integer from 1 to 3; **Y** - identical or different substituents selected from lower alkyl or lower alkoxy; or two vicinal **Y** are connected to each other so that they form a structure (III): 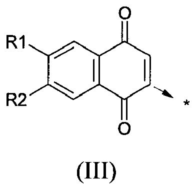 and/or reduced form thereof
wherein R₁ and R₂ are identical or different substituents selected from lower alkyl or lower alkoxy;
**L** - linker group, comprising:
a) either straight or branched hydrocarbon chain optionally containing one or more double or triple bond, or ether bond, or ester bond, or C-S, or S-S, or peptide bond; and which is optionally substituted by one or more substituents preferably selected from alkyl, alkoxy, halogen, keto group, amino group;
b) or natural isoprene chain;
**B** - targeting group comprising:
a) either Skulachev-ion Sk:
Sk⁺ Z⁻
wherein Sk - lipophilic cation;
Z - pharmaceutically acceptable anion;
b) or amphiphilic zwitterion which is able to penetrate into mitochondria in its cationic form; Sk⁺ as a component of **B** may also be a lipophilic metal-organic compound, in particular, lipophilic metalloporphyrin preferably having a structure: 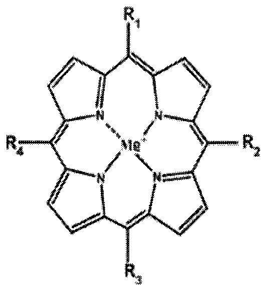
included in the composition of a compound of formula (I) through the moieties designated R₁, R₂, R₃ or R₄, the remaining substituents R₁, R₂, R₃ or R₄ may be selected in accordance with required properties of the compound, for example, in order to increase or decrease hydrophobicity of the molecule; Me⁺ denotes a metal ion preferably selected from Mn, Fe, Co, Cu, Mg or Zn;
produced using at least one of the procedures claimed in claims 1, 3, 6, 7, 9 and that meets the pharmacopoeia requirements.
